(19) Europäisches Patentamt
European Patent Office
Office européen des brevets

(11) EP 1 302 540 A2

(12) **EUROPEAN PATENT APPLICATION**

(43) Date of publication:
**16.04.2003 Bulletin 2003/16**

(51) Int Cl.[7]: **C12N 15/10**, C12N 15/70

(21) Application number: **02021507.5**

(22) Date of filing: **02.02.1998**

(84) Designated Contracting States:
**AT BE CH DE DK ES FI FR GB GR IE IT LI LU MC NL PT SE**

(30) Priority: **31.01.1997 EP 97101539**

(62) Document number(s) of the earlier application(s) in accordance with Art. 76 EPC:
**98910631.5 / 0 968 283**

(71) Applicant: **Cosmix Molecular Biologicals GmbH**
**38124 Braunschweig (DE)**

(72) Inventors:
- **Collins, John**
  **38100 Braunschweig (DE)**
- **Röttgen, Peter**
  **38124 Braunschweig (DE)**

(74) Representative:
**König, Gregor Sebastian, Dipl.-Biol. et al**
**König-Szynka-von Renesse**
**Patentanwälte**
**Lohengrinstrasse 11**
**40549 Düsseldorf (DE)**

Remarks:
This application was filed on 26 - 09 - 2002 as a divisional application to the application mentioned under INID code 62.

# (54) Generation of diversity in combinatorial libraries

(57) The invention concerns gene banks and combinatorial derivatives thereof, prepared using phagemid- or phage-display in combination with type IIS restriction enzymes and cosmid packaging; their use for the isolation of ligands, including enzyme inhibitors, agonists and antagonists for receptors, competitive binding peptides to a defined target, diagnostic ligands for diseases and autoimmune syndromes, including surveillance tools for immune status, post-translationally modified peptides, and such ligands generated by this technology.

EP 1 302 540 A2

## Description

Biotech evolutionary methods, including combinatorial libraries and phage-display technology (PARMLEY & SMITH 1988; SCOTT & SMITH 1990; SMITH 1993), are used in the search for novel ligands of diagnostic, biomedical and pharmaceutical use (reviews; CORTESE 1996; COLLINS 1997). These methods, which use empirical procedures to select molecules with required characteristics, e.g. binding properties, from large populations of variant gene products has been compared to the process of natural evolution. Evolution includes the generation of mutation, selection of functionality over a time period and the ability of the systems to self-replicate. In particular natural systems use recombination to reassort mutations accumulated in the selected population to exponentially increase the combinations of mutations and thus increase the number of variants in the population. This latter aspect, namely the introduction of recombination within mutant genes has only recently been applied to biotech evolutionary methods, although it has been used to increase the size of initial phage-display libraries (e.g. WATERHOUSE 1993; TSURUSHITA 1996; SODOYER 1994; FISCH 1996). STEMMER 1994a, 1994b and 1995 teach that recombination amongst a population of DNA molecules can be acheived in vitro by PCR amplification of a mixture of small overlapping fragments with (1994a, 1994b) or without (STEMMER 1995) primer oligonucleotide sequences being used to drive the PCR reaction. The method is not applicable to recombination within a fully randomized (highly mutated) sequence since the method relies on high homology of the overlapping sequences at the site of recombination. STEMMER 1994b and CRAMERI 1996a do, however, demonstrate the usefulness of in vitro recombination for molecular evolution, where CRAMERI 1996b also demonstrate the use of the method in conjunction with phage-display, even though their method is confined to regions of low mutant density ( ca. 0.5-1% of the bases are mutated in their method) as they state "the advantages of recombination over existing mutagenesis methods are likely to increase with the numbers of cycles of molecular evolution (STEMMER 1994b). We point out that this is due to the self-evident fact that the number of variants created by mutagenesis introducing base changes in existing mutant structures is an additive i.e., a linearly increasing function, whereas the use of recombination between mutated variants yields novel variants as an exponential function of the initial number of variants. The classical phage-display libraries are thus at a grave disadvantage for the generation of novel variants; e.g. to encompass all the possible variants of an octapeptide sequence $20^8$=2.56 x $10^{10}$ different variants would be required.

MARKS 1992 state the importance of recombination in the generation of higher specificity in combinatorial libraries e.g. in attaining antibodies of higher specificity and binding constants in the form of reshuffling light and heavy chains of immunoglobulins displayed in phage-display libraries. These authors do not instruct how the shuffling of all the light and heavy chains in a population heterogeneous in both chains can be acheived, e.g. by a vector allowing recombination. Heavy and light chains were selected one after the other, i.e. an optimal heavy chain first selected from a heterogenous heavy chain population in the presence of a constant light chain, then by preparing a new library, an optimal light chain in combination with the preselected optimal heavy chain. The extensive time consuming sequential optimization strategies currently utilized including consensus-mutational libraries, in vivo mutagenesis, error-pone PCR as well as chain shuffling are summarized in Figures 5 and 6 of COLLINS 1997.

### General background to phage and phage-display libraries

Gene libraries are generated containing extremely large number ($10^6$ to $10^{10}$) of variants. The variant gene segments are fused to a coat protein gene of a filamentous bacteriophage (e.g. M13, fd or f1), and the fusion gene is inserted into the genome of the phage or of a phagemid. A phagemid is defined as a plasmid containing the packaging and replication origin of the filamentous bacteriophage. This latter property allows the packaging of the phagemid genome into a phage coat when it is present in an *Escherichia coli* host strain infected with a filamentous phage (superinfection). The packaged particles produced, be they phage or phagemid, display the fusion protein on the surface of the particles secreted into the medium. Such packaged particles are able to inject their genomes into a new host bacterium, where they can be propogated as phage or plasmids, respectively. The special property of the system lies in the the fact that since the packaging takes place in individual cells usually infected by a single variant phage/ phagemid, the particles produced on propogation contain the gene encoding the particular variant displayed on the particle's surface. Several cycles of affinity selection for clones exhibiting the required properties due to the particular property of the variant protein displayed, e.g. binding to a particular target molecule immobilized on a surface, followed by amplification of the enriched clones leads to the isolation of a small number of different clones having these properties. The primary structure of these variants can then be rapidly elucidated by sequencing the hypermutated segment of the variant gene.

### Efficiency of producing combinatorial libraries

There are a number of factors which limit the potential of this technology. The first is the number and diversity of the variants which can be generated in the primary library. Most libraries have been generated by transformation of ligated DNA preparations into *Escherichia coli* by electroporation. This gives an efficiency of ca. 0.1 to 1x $10^6$ recombinants /microgram ligated phage DNA. The highest cloning efficiency reported (of $10^7$ recombinants per microgram insert DNA) is obtained using special lambda vectors into which a single filamentous phage vector is inserted, in a special cloning site, bracketted by a duplication of the filamentous phage replication/packaging origin (AMBERG 1993; HOGREFE 1993a+b). The DNA construct is efficiently introduced into the *Escherichia coli* host after packaging into a lambda bacteriophage coat in an *in vitro* lambda packaging mix. Infection of a strain carrying such a hybrid phagemid by an M13- helper phage allows excision and secretion of the insert packed in a filamentous phage coat. Neither AMBERG 1993 nor HOGREFE 1993a+b instruct on how the method may be used to introduce recombination during this procedure. Although they mention that the efficiency may be improved by the use of type IIs restriction endonucleases during the construction of the concatemers used as substrate for the in vitro packageing no examples are given and in the ensuing five years no examples have appeared in the literature. The procedure described in our invention also uses the high efficiency of the invitro lambda packaging , but maximizes the capacity of the cloning vector by using a cosmid vector (8) in which many copies (say 8) of the phagemid are inserted in each construct. One of the surprising innovative aspects of this procedure is the discovery of a number of protocols for the de novo synthesis of large hypervariable libraries. One type is particularly efficient, in that phagemid/cosmid vectors are forced to integrate into the hybrid concatamers oriented in the same orientation. Any variant of the protocol which does not ensure this feature does not work efficiently.

**The use of type IIs restriction endonucleases**

SZYBALSKI 1991 teaches a large number of novel applications for type IIs restriction endonucleases, including precise trimming of DNA, retrieval of cloned DNA, gene assembly, use as a universal restriction enzyme, cleavage of single-stranded DNA, detection of point mutations, tandem amplification, printing amplification reactions and localisation of methylated bases. They do not give any instruction as to how such enzymes can be used in the creation of recombination within highly mutated regions, e.g. within a combinatorial library.

**Reference list**

Amberg, J, Hogrefe, H., Lovejoy, H., Hay, B., Shopes, B, Mullinax, R. and Sorge, J.A.(1993), Strategies, 5, 2-3.
Collins, J. (1997) Phage display. In Moos, W.H. et al. (eds) Annual reports in combinatorial chemistry and molecular diversity. Vol. 1., ESCOM Science publ., Leiden. pp. 210-262. Cortese, R.(ed.) (1996) Combinatorial libraries: Synthesis, Screening and Application potential. Walter de Gruyter, Berlin. Crameri, A., Whitehorn, E.A., Tate, E. and Stemmer, W.P.C.(1996a) 14, 315-319
Crameri, A., Cwirla, S. and Stemmer, W.P.C.(1996b) Nat. Med. 2, pg.100
Fisch, I., Kontermann, R. E., Finnern, R., Hartley, O., Soler-Gonzalez, A. S., Griffiths, A. D. and Winter, G. (1996) Proc. Natn. Acad. Sci. USA. 93, 7761.
Marks, J. D.; Griffiths, A. D.; Malmqvist, M.; Clackson, T. P.; Bye, J. M. and Winter, G. (1992) BioTechnol. 10, 779-783.
Hogrefe, H. H., Amberg, J. R., Hay, B. N., Sorge, J. A. and
Shopes, B. (1993) Gene, 137, 85-91.
Hogrefe, H. H., Mullinax, R. L., Lovejoy, A.E., Hay, B. N. and
Sorge, J.A.(1993) Gene 128, 119-126
Parmley, S. F. and Smith, G. P. (1988) Gene 73, 305- 318
Scott, J. K. and Smith, G. P. (1990) Science 249, 386-390
Smith, G. P. (1993) Gene 128, 1-2.
Sodoyer, R., Aujume, L., Geoffrey, F., Pion, C., Puebez, I., Montegue, B., Jacquemot, P. and Dubayle, J. (1996) In Kay, B. K. et al. (eds.) Phage display of peptides and proteins. A laboratory manual. Academic Press, San Diego. Pp. 215-226
Stemmer, W.P.C. (1994a) Nature (Lond.) 370, 389-391
Stemmer, W.P.C. (1994b) Proc.Nat..Acad Sci.USA, 91, 10747-10751
Stemmer , W.P.C (1995) Gene 164, 49-53
Szybalski, W., Kim, S. C., Hasan, N. and Podhajska, A. J. (1991) Gene, 100, 13-26.
Tsurushita, M., Fu, H. and Warren, C. (1996) Gene, 172, 59.
Waterhouse, P. , Griffiths, A. D., Johnson, K. S. and Winter, G. (1993a) Nucleic Acid Res. 2265-2269

According to a first embodiment the invention concerns a bank of genes, wherein said genes comprise a double stranded DNA sequence which is represented by the following formula of one of their strands:

$$5'B_1B_2B_3...B_nX_{n+1}...X_{n+a}Z_{n+a+1}Z_{n+a+2}X_{n+a+3}...X_{n+a+b}Q_{n+a+b+\ 1}...Q_{n+a+b+j}3'$$

wherein n, a, b and j are integers and
$n > 3$, $a > 1$, $b > 3$ and $j > 1$,
wherein $X_{n+1}...X_{n+a+b}$ is a hypervariable sequence and B, X, Z and Q represent adenine (A), cytosine (C), guanine (G) or thymine (T),

(i) Z represents G or T at a G:T ratio of about 1:1, and/or
(ii) Z represents C or T at a C:T ratio of about 1:1, and/or

laboratory manual. Academic Press, San Diego. Pp. 215-226 Stemmer, W.P.C. (1994a) Nature (Lond.) 370, 389-391 Stemmer, W.P.C. (1994b) Proc.Nat..Acad Sci.USA, 91, 10747-10751 Stemmer , W.P.C (1995) Gene 164, 49-53 Szybalski, W., Kim, S. C., Hasan, N. and Podhajska, A. J. (1991) Gene, 100, 13-26.
Tsurushita, M., Fu, H. and Warren, C. (1996) Gene, 172, 59. Waterhouse, P. , Griffiths, A. D., Johnson, K. S. and Winter, G. (1993a) Nucleic Acid Res. 2265-2269

According to a first embodiment the invention concerns a bank of genes, wherein said genes comprise a double stranded DNA sequence which is represented by the following formula of one of their strands:

$$5'B_1B_2B_3...B_nX_{n+1}...X_{n+a}Z_{n+a+1}Z_{n+a+2}X_{n+a+3}...X_{n+a+b}Q_{n+a+b+\ 1}...Q_{n+a+b+j}3'$$

wherein n, a, b and j are integers and
$n > 3$, $a > 1$, $b > 3$ and $j > 1$,
wherein $X_{n+1}...X_{n+a+b}$ is a hypervariable sequence and B, X, Z and Q represent adenine (A), cytosine (C), guanine (G) or thymine (T),

(i) Z represents G or T at a G:T ratio of about 1:1, and/or

(ii) Z represents C or T at a C:T ratio of about 1:1, and/or

(iii) Z represents A or G at a A:G ratio of about 1:1, and/or

(iv) Z represents A or C at a A:C ratio of about 1:1, and wherein

subsequences $B_1...B_n$ and/or $Q_{n+a+b+1}...Q_{n+a+b+j}$ represent recognition sites for restriction enzymes, and wherein the recognition sites are orientated such that their cleavage site upon cleavage generates a cohesive end including the two bases designated Z.

Restriction of this sequence with a type IIs restriction enzyme as thus described, followed by religation leads to the recombination of the hypervariable regions located 5' and 3' of the cleavage site. This is the essence of the methodology which we designate "cosmix-plexing". It is essential in this procedure that the fragments generated on cleavage by the restriction enzyme are religated in the correct orientation ("head-to-tail"), whereby the Z sequences are chosen for the four libraries ((i) to iv)) so as to ensure this (see below) yet still allowing all possible amino-acids to be encoded at the cleavage site. If this correct orientation is not ensured there will be a drastic reduction in both the percent of correctly reconstituted fusion-protein genes, a reduction in the proportion of molecules which can be packaged in vitro in the lambda-packaging extracts (which requires the correct orientation of the cos-sites), as well as a reduction in the proportion of in vivo excisable phagemid copies from the cosmid concatemer ( excision requires the correct orientation of consecutive phage replication origins). correct orientation correct orientation incorrect orientation (head-to-head ligation)

```
5'------>XGG/x----->          -------->XCC/x---->      -------
>XGG/Y---->
3'--------Y/CCy< -----        ----------Y/GGy< ---          ----
-----Y/CCX< ---
```

To prevent the problems arising from false orientation (head-to-head) mentioned in the previous paragraph, the four gene libraries mentioned in claim must be kept separated during cosmix-plexing. In fact with respect to the formation of recombinants the libraries behave as 16 separate sets which cannot recombine with each other: four libraries maintained separately, where each set contains four possible cohesive ends, e.g. library (i) with Z= G or T contains:

```
5' ---->XGT/Y---->,   ----->XGG/Y----->,   ---->XTG/Y---->,
and  ---->XTT/Y----->
3' ------y/CA x< --      -------y/CC x< -----    -----y/AC x< -
---               ------y/AA x< ----
```

It is evident that problems of false orientation will arise on mixing the different libraries, e.g. the "AC library (iv) will contain AA, AC, CA and CC sequences which can pair in the false orientation with, respectively each of the cohesive ends generated in library (i).

A specific embodiment of the invention concerns a bank of genes wherein subsequences $B_1 ... B_n$ or $Q_{n+a+b+1} ... Q_{n+a+b+j}$ represent recognition sites for restriction enzymes and wherein the recognition sites are orientated such that their cleavage site upon cleavage generates a cohesive end including the two bases designated Z.

Further, a specific embodiment concerns a bank of genes, wherein the cohesive end is a 2 bp single strand end formed by the two bases designated Z.

Further, a specific embodiment concerns a bank of genes wherein each gene is provided as display vector, especially as M13 phage or M13 like phage or as phagemid.

Another embodiment of the invention concerns a set of four gene banks according to the invention wherein the gene banks are characterized as follows:

- first gene bank: Z represents G or T, preferentially at a G:T ratio of about 1:1;
- second gene bank: Z represents C or T, preferantially at a C:T ratio of about 1:1;
- third gene bank: Z represents A or G, preferentially at a A:G ratio of about 1:1; and
- fourth gene bank: Z represents A or C, preferentially at a A:C ratio of about 1:1.

A specific embodiment of the invention concerns a set of four gene banks wherein each gene is provided as display vector, especially as M13 phage or M13 like phage or as phagemid.

Another embodiment of the invention concerns a bank of genes wherein said genes comprise a double stranded DNA sequence which is represented by the following formula of one of their strands:

$$5'B_1B_2B_3...B_nX_{n+1}...X_{n+a}Z_{n+a+1}Z_{n+a+2}X_{n+a+3}...X_{n+a+b}Q_{n+a+b+\ 1}...Q_{n+a+b+j}3'$$

wherein n, a, b and j are integers and
$n > 3$, $a > 1$, $b > 3$ and $i > 1$,

wherein $X_{n+1}...X_{n+a+b}$ is a hypervariable sequence and B, X, Z and Q represent adenine (A), cytosine (C), guanine (G) or thymine (T), and wherein

four sets of oligonucleotide sequences comprising $Z_{n+a+1}$ and $Z_{n+a+2}$ are present, preferentially at a ratio of (i):

(ii):(iii):(iv) of about 1:1:2:2, wherein the four sets are characterized as follows:

first set: $Z_{n+a+1}$ represents G and $Z_{n+a+2}$ also represents G;

second set: $Z_{n+a+1}$ represents C and $Z_{n+a+2}$ represents T;

third set: $Z_{n+a+1}$ represents A and $Z_{n+a+2}$ represents A or C, preferentially at A:C ratio of about 1:1; and

fourth set: $Z_{n+a+1}$ represents T and $Z_{n+a+2}$ represents C or G, preferentially at a C:G ratio of about 1:1, and wherein sequences $B_1 ... B_n$ and/or $Q_{n+a+b+1} ... Q_{n+a+b+j}$ represent recognition sites for restriction enzymes, wherein the recognition sites are orientated such that their cleavage site upon cleavage generates a cohesive end including the two bases designated Z.

A specific embodiment of the invention concerns a bank of genes wherein the four sets of oligonucleotide sequences are present at a ratio of (i):(ii):(iii):(iv) of (0 to 1):(0 to 1):(0 to 1):(0 to 1) with the proviso that at least one of said sets is present.

Further, a specific embodiment of the invention concerns a bank of genes wherein subsequences $B_1 ... B_n$ and/or $Q_{n+a+b+1} ... Q_{n+a+b+j}$ represent recognition sites for restriction enzymes and wherein the recognition sites are orientated such that their cleavage site upon cleavage generates a cohesive end including the two bases designated Z.

Furhter, a specific embodiment of the invention concerns a bank of genes wherein the cohesive end is a 2 bp single strand end formed by the two bases designated Z.

Another embodiment of the invention concerns bank of genes wherein said genes comprise a double stranded DNA sequence which is represented by the following formular of one of their strands:

$$5'B_1B_2B_3 ... B_nX_{n+1} ... X_{n+a}Z_{n+a+1}Z_{n+a+2}X_{n+a+3} ... X_{n+a+b}Q_{n+a+b+}\ 1\ ... Q_{n+a+b+j}3'$$

wherein n, a, b and j are integers and
n > 3, a > 1, b > 3 and j > 1,
wherein $X_{n+1} ... X_{n+a+b}$ is a hypervariable sequence and B, X, Z and Q represent adenine (A), cytosine (C), guanine (G) or thymine (T), and wherein
the following six sets of oligonucleotide sequences comprising $X_{n+a}$, $Z_{n+a+1}$ and $Z_{n+a+2}$ are present, preferably at a ratio of (i):(ii):(iii):(iv):(v):(vi) of about 3:4:3:4:4:1, wherein the six sets are characterized as follows:

first set: $X_{n+a}$ represents A, G and/or T, preferentially at a ratio of about 1:1:1 or $X_{n+a}$ represents C, G and/or T, preferentially at a ratio of about 1:1:1, $Z_{n+a+1}$ represents G and $Z_{n+a+2}$ represents G;

second set: $X_{n+a}$ represents A, C, G and/or T, preferentially at a ratio of about 1:1:1:1, $Z_{n+a+1}$ represents C and $Z_{n+a+2}$ represents T;

third set: $X_{n+a}$ represents A, C and/or G, preferentially at a ratio of about 1:1:1, $Z_{n+a+1}$ represents A and $Z_{n+a+2}$ represents A;

fourth set: $X_{n+a}$ represents A, C, G and/or T, preferentially at a ratio of about 1:1:1:1, $Z_{n+a+1}$ represents A and $Z_{n+a+2}$ represents C;

fifth set: $X_{n+a}$ represents A, C, G and/or T, preferentially at a ratio of about 1:1:1:1, $Z_{n+a+1}$ represents T and $Z_{n+a+2}$ represents C;

sixth set: $X_{n+a}$ represents A, $Z_{n+a+1}$ represents T and $Z_{n+a+2}$ represents G.

**Single-tube" method**

**Problem**

A method should be developed which allows cosmix-plexing without maintaining separate libraries. This would have the advantage of reducing manipulation, involved in screening the four separate libraries, as previously described.

This would offer a saving in both time and materials. This has been acheived in two separate versions of the invention.

**Solution**

It is possible to select combinations of nucleotides within the cohesive ends generated by type IIs restriction within the aforementioned sequence, i.e. ZZ, in which all the clones are present in a single library and in which the possibility of false orientation during ligation, and the associated loss of efficiency associated with this, is eliminated. At the same time the number of subsets, defined by the number of different cohesive ends which can be generated, which cannot interact (recombine) with each other, is reduced from the 16 sets, as in the previously described version of the method, to 6.

**Designing the sequences**

The combinations of 2 bp single-strand cohesive end sequences which can be generated at ZZ are theoretically as follows:

| | | | |
|---|---|---|---|
| **AA** | **CA** | **GA** | TA |
| **AC** | **CC** | GC | **TC** |
| **AG** | CG | **GG** | **TG** |
| AT | **CT** | **GT** | **TT** |

Of these, the sequences with an inverted symmetry axis (palindromes: AT, TA, GC, CG ), can pair in both orientations and are thus to be eliminated from cosmix-plexing libraries for the reasons given above. The remaining 12 sequences are actually 6 sets of complementary pairs ( e.g. CC+GG, AA+TT, CA+TG). By choosing one partner from each pair (total of 6) a single set of cohesive ends can be generated which can pair only in the correct "head-to-tail" orientation. The actual choice of sequences takes the codon usage into account, assuming that ZZ are chosen as the 2nd and 3rd position of the codon. Determining are the amino-acids which are encoded by either a single or only two codons( single codon methionine (**TG**) and tryptophan (**GG**); after elimination of the palindromic sequences there also only single codons available encoding aspartic acid (Asp), asparagine (Asn), cystine (Cys), histidine (His) and tyrosine (Tyr). To encode Asp, Asn, His and Tyr an **AC** sequence is required. Selecting **AC** has the default that the complimentary sequence **GT** must be avoided. This is the only possibility of encoding Cys. However, the inclusion of Cys within the hypervariable sequence often causes problems of misfolding and the formation of dimeric aggregates, dependent on the redox potential of the environment. It was thus decided to create a set in which Cys codons are eliminated, but which will be of great use in many applications, including cyclic peptide library formation. If the sequence **AA** is chosen to encode glutamic acid (Glu), glutamine (Gln) and lysine (Lys) also allowing the stop-codon TAA, then TT must be eliminated. The consequence of this is that **TC** must also be included so that phenylalanine (Phe) and isoleucine (Ile) can be encoded. The elimination of the complimentary GA is without consequence since other GG codon(s) encode argenine (Arg) and glycine (Gly). The elimination of CC is then without consequence, since alanine (Ala), proline (Pro), serine (Ser) and threonine (Thr) can be encoded by **CT**-containing codons. This is the argumentation for the selection of ZZ sequences designated "combination A" below.

For the sake of completeness: if the doublet **AA** were left out and, consequently **TT** included, then **AG** must be included to encode Glu, Gln and Lys. In order to encode Ala and Pro, either CT (combination B) or CA (combination C) must now be included. This leads to the inclusion of either AG and CT (combi. B), or CA and TG (combi. C) as complimentary pairs. Combinations B and C thus do not represent an adequate solution to the problem.

| combination A | | combination B | | combination C | |
|---|---|---|---|---|---|
| **AA** | TT | AA | **TT** | AA | **TT** |
| **AC** | GT | **AC** | GT | **AC** | GT |
| AG | **CT** | **AG** | **CT** | **AG** | CT |
| CA | **TG** | CA | **TG** | **CA** | **TG** |
| CC | **GG** | CC | **GG** | CC | GG |
| GA | **TC** | **GA** | TC | **GA** | **TC** |

Sequences chosen are shown in bold type. Complementary pairs are adjacent to each other.

Table 1: Genetic code; the selection of XZZ codons used according to combination A is shown in bold type.

| Ala | Arg | Asp | Asn | Cys | Glu | Gln | Gly | His | Ile | Leu |
|---|---|---|---|---|---|---|---|---|---|---|
| GCA | AGA | **GAC** | **AAC** | TGC | **GAA** | **CAA** | GGA | **CAC** | ATA | TTA |
| GCC | **AGG** | GAT | AAT | TGT | GAG | CAG | GGC | CAT | **ATC** | **TTG** |
| GCG | CGA | | | | | | **GGG** | | ATT | CTA |
| **GCT** | CGC | | | | | | GGT | | | **CTC** |
| | **CGG** | | | | | | | | | **CTG** |
| | CGT | | | | | | | | | CTT |

| Lys | Met | Phe | Pro | Ser | Thr | Trp | Tyr | Val | Stop |
|---|---|---|---|---|---|---|---|---|---|
| **AAA** | **ATG** | **TTC** | CCA | AGC | ACA | **TGG** | **TAC** | GTA | **TAA** |
| AAG | | TTT | CCC | AGT | ACC | | TAT | **GTC** | TAG |
| | | | CCG | TCA | ACG | | | **GTG** | TGA |
| | | | **CCT** | TCC | **ACT** | | | GTT | |
| | | | | TCG | | | | | |
| | | | | **TCT** | | | | | |

Table 2. Frequency of the amino-acids, comparing the selected combination A (above) and the natural frequency of all codons.

| | Amino-acid | natural frequency | Combination A |
|---|---|---|---|
| | Ala | 4 | 1 |
| | Arg | 6 | 2 |
| | Asp | 2 | 1 |
| | Asn | 2 | 1 |
| | Cys | 2 | 0 |
| | Glu | 2 | 1 |
| | Gln | 2 | 1 |
| | Gly | 4 | 1 |
| | His | 2 | 1 |
| | Ile | 3 | 1 |
| | Leu | 6 | 3 |
| | Lys | 2 | 1 |
| | Met | 1 | 1 |
| | Phe | 2 | 1 |
| | Pro | 4 | 1 |
| | Ser | 6 | 1 |
| | Thr | 4 | 1 |
| | Trp | 1 | 1 |
| | Tyr | 2 | 1 |
| | Val | 4 | 2 |
| | Stop | 3 | 1 |
| Total | 21 | 64 | 24 |

**Creation of a set of four oligonucleotides according to combination A**

Gene libraries can be created according the requirements of the combination A, by creating four sets of nucleotides in which $X_{n+a}Z_{n+a+1}Z_{n+a+2}$ are:

i) NGG
ii) NCT
iii) NA(A or C)
iv). NT (C or G),

where N is C,G,A or T.

After the synthesis of these oligonucleotides they can be combined to obtain a single-tube cosmix-plexing gene library, whereby to obtain the relative codon frequencies given in Table 2 the gene libraries i) to iv) are present in the final mixture at a ratio of 1:1:2: 2, respectively. As explained above this mixture will always give a correct orientation on religation of type IIs restriction enzyme-cleaved fragments having the 2bp single-stranded cohesive ends ZZ.

**Alternatively: a set of six oligonucleotides conforming to combination A**

Gene libraries can be created according a modification of combination A, in which both Stop and cystine codons are eliminated, and in which each of the other amino-acids is each represented by a single codon, by creating six sets of nucleotides in which $X_{n+a}Z_{n+a+1}Z_{n+a+2}$ are:

i) (A, G or T)GG or (C,G or T)GG
ii) NCT
iii) (A, G or C)AA
iv) NAC
v) NTC
vi) ATG

After the synthesis of these oligonucleotides they can be combined to obtain a single-tube cosmix-plexing gene library, whereby to obtain the equimolar codon frequencies for each amino-acid the gene libraries i) to vi) are present in the final mixture at a ratio of 3: 4: 3: 4: 4: 4: 1 respectively. As explained above this mixture will always give a correct orientation on religation of type IIs restriction enzyme-cleaved fragments having the 2bp single-stranded cohesive ends ZZ.
Again, as with the previous sets this single-tube library represents six-subsets which are unable to recombine with each other during cosmix-plexing.
*** the following section has been altered radically. The last Tables are no longer necessary. **Consideration of the central amino-acid codon created during cosmix-plexing recombination**
The amino-acid at the recombination site is determined by the 5'-hypervariable segment. The set of amino-acids which may be represented at this position is defined for each subset as presented in Table 2.

**Consideration of the number of clones needed in a "representative" library**

The minimal number of clones required in a library to include all possible amino-acid sequences in a random peptide containing 'n' amino-acids is $20^n$, i.e. for n=9, $20^9$= 5.12 x $10^{11}$. In fact, at a confidence limit of say 95%, this figure must be some three-fold higher, to allow for the statistics of sampling, i.e. ca. 1.5 x $10^{12}$. In practice this figure may be higher due to, e.g. non-random synthesis of the oligonucleotides used to generate the library as well as biased codon representation (for a detailed discussion see Collins 1997).

**Consideration of the number of recombined clones generated by cosmix-plexing**

The cosmix-plexing strategy is based on the concept that in initial selection experiments clone populations will be enriched for sequences which contain structural elements based on the primary sequence in the varied segment. Even if the optimal sequence is not present due to the limitations imposed by the limited size of the initial library, cosmix-plexing will increase the likelihood of finding just such a sequence by providing a large number of novel recombinants in which the 5'- and 3'-"halves" of the varied section are reassorted e.g. for the hypervariable nonapeptide library described in the example, the sequences encoding the amino-proximal five amino acids are recombined with the sequences encoding the carboxy-proximal four amino-acids. Since the cohesive ends essentially limit the recombination to defined subsets, in which one subset cannot undergo recombination with any of the other subsets, the actual number of recombinants generated is less than could be obtained with completely random recombination.
For the initial four-tube protocol described, four separate libraries each containing four subsets are used:
Random recombination would generate, for a set of N clones, $N^2$ recombinants, assuming $N^2$ is less than or equal to the theoretical number of variants ($20^n$, see above) which can be encoded within the hypervariable segment, otherwise it will tend to $20^n$.
For the four-tube protocol 16 subsets are created each representing a pool within which recombination can take place. If the total the library consists of N clones then the number of novel recombinants which can be formed within each of the 16 subsets is $(N/16)^2$. Summing for all sixteen subsets, the number of recombinants which can be generated is 16 x $(N/16)^2$ = $N^2/16$, again assuming $N^2/16$ is less than or equal to the theoretical number of variants ($20^n$, see above) which can be encoded within the hypervariable segment, otherwise it will tend to $20^n$.
For the single-tube protocol only 6 subsets are created. each representing a pool within which recombination can take place. If the total library consists of N clones then the number of novel recombinants which can be formed within each of the 6 subsets is $(N/6)^2$. Summing for all six subsets, the number of recombinants which can be generated is 6 x $(N/6)^2$= $N^2/6$, again assuming $N^2/6$ is less than or equal to the theoretical number of variants ($20^n$, see above) which can be encoded within the hypervariable segment, otherwise it will tend to $20^n$.
It is thus clear that the single-tube version of the invention is superior not only in terms of time and economy of the procedure but in the potential to generate a greater diversity from a given number of clones during cosmix-plexing guided recombination.

A specific embodiment of the invention concerns a bank of genes, wherein the six sets of oligonucleotide sequences are present at a ratio of (i):(ii):(iii):(iv):(v):(vi) of (0 to 1):(0 to 1):(0 to 1):(0 to 1):(0 to 1):(0 to 1) with the proviso that at least one of said sets is present.

Further, a specific embodiment of the invention concerns a bank of genes wherein each gene is provided as display vector, especially as M13 phage or M13 like phage or as phagemid.

Further, a specific embodiment of the invention concerns a bank of genes wherein the double stranded DNA sequence is comprised by a DNA region (fusB) encoding a peptide or a protein to be displayed.

Further, a specific embodiment of the invention concerns a bank of genes, **characterized** in that n = j = 6, a = 14 and b = 16.

Further, a specific embodiment of the invention concerns a bank of genes wherein the restriction enzyme is a tyoe IIS restriction enzyme.

Further, a specific embodiment of the invention concerns a bank of genes which is **characterized** in that

(a) subsequence $B_1...B_n$ is the recognition site for the restriction enzyme BpmI (CTGGAG) and subsequence $Q_{n+a+b+1}...Q_{n+a+b+j}$ is an inverted BsgI recognition site (CTGCAC); or

(b) subsequence $B_1...B_n$ is the recognition site for the re-striction enzyme BsgI (GTGCAG) and subsequence $Q_{n+a+b+1}...Q_{n+a+b+j}$ is an inverted BpmI recognition site (CTCCAG).

Further, a specific embodiment of the invention concerns a bank of genes which is characterized in that the hypervariable sequence $X_{n+1}... X_{n+a+b}$ contains NNB or NNK wherein N = adenine (A), cytoseine (C), guanine (G) or thymine (T) ;
B = cytosine (C), guanine (G) or thymine (T); and
K = guanine (G) or thymine (T).

Another embodiment of the invention concerns a phagemid pROCOS4/7 of the sequence shown in **Fig. 6.**

Still another embodiment of the invention concerns a phagemid pROCOS5/3 of the sequence shown in **Fig. 7.**

Another embodiment of the invention concerns a method for the production of large

- phage-display libraries or
- phagemid-display libraries,

containing or consisting of optionally packaged recombined display vectors, wherein recombination takes place at the cleavage site(s) for a restriction enzyme (cut (B) enzyme; arrow in **Fig. 3**) and wherein

(a) to (b) a double-stranded DNA prepared from Escherichia coli cells containing a display vector population, consisting of M13 phages or M13 like phages or consisting of phagemids according to the invention; a cosmid vector; a restriction enzyme for cut (B); and a restriction enzyme for cut (A) are selected, wherein

(i) the cut (B) enzyme cleaves the display vectors in the region encoding the displayed peptide or displayed protein (arrow in **Fig. 3**) and generates unique non-symmetrical cohesive ends, wherein each cohesive end is a 2 bp single strand end formed by the two bases designated Z, and

(ii) the cut (A) enzyme cleaves the display vectors and the cosmid vector and generates upon cleavage unique non-symmetrical cohesive ends (fusA) which differ from those resulting from cut (B),

(c) the display vectors are cleaved with the first restriction enzyme,

(d) the display vector and the cosmid vector are cleaved with the second restriction enzyme,

(e) the cleaved display vectors are ligated with the cleaved cosmid vectors forming concatamers,

(f) the ligation product is subjected to a lambda packaging and transduced into an Escherichia coli host,

(g) if wanted, selection is made for a gene present in the ligated display vectors,

(h) the transduced display vectors in the Escherichia coli host are

- either in the case of a phage-display vector spontaneously packaged in M13 or M13 like phage coats
- or in the case of a phagemid-display vector packaged by infecting the Escherichia coli host with an M13 type helper phage (superinfection),

(i) the packaged display vectors are passaged in a fresh Escherichia coli host and phage-display or phagemid-display libraries are formed and, if wanted,

(j) the passaged display vectors are

- either in the case of a phage-display vector spontaneously packaged in M13 or M13 like phage coats
- or in the case of a phagemid-display vector packaged by infecting the fresh Escherichia coli host with an M13 type helper phage (superinfection) and

phage-display or phagemid-display libraries are formed.

A specific embodiment of the invention concerns a method which is **characterized** in that in steps (a) to (b) a type IIS restriction enzyme is selected, preferably BglI, DraIII, BsgI or BpmI.

Further, a specific embodiment of the invention concerns a method which is **characterized** in that for cuts (B) and (A) the same restriction and/or restriction enzyme is selected.

Further, a specific embodiment of the invention concerns a method which is **characterized** in that as cut (B) enzyme and as cut (A) enzyme different enzymes are used (**Fig. 3**), preferably BsgI or BpmI as cut (B) enzyme and DraIII as cut (A) enzyme (fd or M13 replication origin cut).

Further, a specific embodiment of the inven tion concerns a method which is characterized **characterized** in that in step (h) and facultatively in step (j) M13K07 is used as M13 type helper phage.

Further, a specific embodiment of the invention concerns a method which is characterized in that **characterized** in that the ophagemid and the cosmid are identical and, further, presence of and cleavage with cut (A) enzyme is optional and/or cut (B) enzyme and cut (A) enzyme are identical .

Further, a specific embodiment of the invention concerns a method which is **characterized** in that in step (i) the multiplicity of infection (MOI) is less than or equal to 1.

Further, a specific embodiment of the invention concerns a method wherein the cosmid comprises an fd or M13 bacteriophage origin (replication/packaging).

Further, a specific embodiment of the invention concerns a method wherein in step (e) a mol ratio of display vectors to the cosmid vector within the range of from 3:1 to 15:1 and preferably 3:1 to 10:1 is used.

Further, a specific embodiment of the invention concerns a method wherein in step (e) a vector concentration (comprising display vectors and cosmid vectors) of more than 100 µg DNA/ml is used.

Another embodiment of the invention concerns a method for the production of large

- phage-display extension libraries or
- phagemid-display extension libraries, wherein

an oligonucleotide cassette of d bases in length is inserted into a restriction site (cut (B)) via the cohesive ends ZZ as defined above to yield a sequence (supra sequence) or a gene comprising a double stranded DNA sequence which is represented by the following formula of one of their strands:

$$5'B_1..B_nX_{n+1}..X_{n+a}Z_{n+a+1}Z_{n+a+2}X_{n+a+3}..X_{n+a+d}Z_{n+a+d+1}Z_{n+a+d+2}X_{n+a+d+3}..X_{n+a+d+b}Q_{n+a+d+b+1}..Q_{n+a+d+b+j}3'$$

wherein d is an integer and a multiple of 3, preferably within the range of from 6 to 36; n, a, b and j and B, X, Z and Q have the same meaning as in any of the preceding claims; and wherein

(a) to (b) a double-stranded DNA prepared from Escherichia coli cells containing a display vector population, consisting of M13 phages or M13 like phages or consisting of phagemids according to the invention; a cosmid vector; a restriction enzyme for cut (B); and a restriction enzyme for cut (A) are selected, wherein

(i) the cut (B) enzyme cleaves the display vectors in the region encoding the displayed peptide or displayed protein and generates unique non-symmetrical cohesive ends; wherein each cohesive end is a 2 bp single strand end formed by the two bases designated Z,

(ii) the cut (A) enzyme cleaves the display vectors and the cosmid vector such that unique non-symmetrical cohesive ends are formed which differ from those resulting from cut (B) ,

(c1) the display vectors are cut with the cut (B) restriction enzyme,

(c2) a DNA cassette is inserted into the cleavage site with their ZZ cohesive ends,

(d) the resulting display vector and the cosmid vector are cleaved with the cut (A) restriction enzyme,

(e) the cleaved display vectors are ligated with the cleaved cosmid vectors forming concatamers,

(f) the ligation product is subjected to a lambda packaging and transduced into an Escherichia coli host such that the DNA cassette lies betweeen two hypervariable sequences (extension sequences),

(g) if wanted, selection is made for a gene present in the ligated display vectors,

(h) the transduced display vectors in the Escherichia coli host are

- either in the case of a phage-display vector spontaneously packaged in M13 or M13 like phage coats
- or in the case of a phagemid-display vector packaged by infecting the Escherichia coli host with an M13 type helper phage (superinfection),

(i) the packaged display vectors are passaged in a fresh Escherichia coli host and phage-display or phagemid-display libraries are formed, and, if wanted,

(j) the passaged display vectors are

- either in the case of a phage-display vector spontaneously packaged in M13 or M13 like phage coats
- or in the case of a phagemid-display vector packaged by infecting the fresh Escherichia coli host with M13 type helper phages (superinfection) and

phage-display or phagemid-display extension libraries are formed.

Another embodiment of the invention concerns a method for the reassortment of the 5'- and/or 3'-extensions in the production of large recombinant

- phage-display extension libraries or
- phagemid-display extension libraries,

comprising the sequence as defined before wherein recombination takes place at one or the other, or consecutively at both the cleavage site(s) ZZ bracketting the inserted cassette(s), wherein

(a) to (b) a double-stranded DNA prepared from Escherichia coli cells containing a display vector population, consisting of M13 phages or M13-like phages or consisting of phagemids as display vectors as defined before; a cosmid vector; a restriction enzyme for cut (B); and restriction enzyme for cut (A) are selected, wherein

(i) the cut (B) enzyme cleaves the display vectors in the region encoding the displayed peptide or displayed protein and generates unique non-symmetrical cohesive ends at selectively either

- the 5'-junction of extension and cassette (cleavage by the restriction enzyme recognizing the binding site $B_1 \ldots B_n$ as defined before), or
- at the 3'-junction of extension and cassette (cleavage by the restriction enzyme recognizing the binding site $Q_{n+a+b+1} \ldots Q_{n+a+b+j}$ as defined before, or $Q_{n+a+d+b+1} \ldots Q_{n+a+d+b+j}$ as defined before), wherein each cohesive end is a 2 bp single strand end formed by the two bases designated Z,

(ii) the cut (A) enzyme cleaves the display vectors and the cosmid vector and generates upon cleavage unique non-symmetrical cohesive ends which differ from those resulting from cut (B),

(b) the display vectors are cleaved with the first restriction enzyme,

(c) the display vector and the cosmid vector are cleaved with the second restriction enzyme,

(e) the cleaved display vectors are ligated with the cleaved cosmid vectors forming concatemers,

(f) the ligation product is subjected to a lambda packaging and transduced into an Escherichia coli host,

(g) if wanted, selection is made for a gene present in the ligated display vectors,

(h) the transduced display vectors in the Escherichia host are

- either in the case of a phage-display vector spontaneously packaged in M13 or M13-like phage coats
- or in the case of phagemid-display vectors packaged by infecting the Escherichia coli host with an M13-type helper bacteriophage (superinfection),

(i) the packaged display vectors are passaged in a fresh Escherichia coli host and phage-display or phagemid-display libraries are formed and, if wanted

(j) the passaged display vectors are

- either in the case of a phage-display vector spontaneously packaged in an M13 or M13-like phage coats
- or in the case of a phagemid vector packaged by infecting the fresh Escherichia coli host with M13 type helper phages (superinfection) and

phage-display or phagemid-display libraries are formed.

A specific embodiment of the invention concerns a method which is characterized in that in steps (a) to (b) a type IIs restriction enzyme is selected, preferably BglI, DraIII, BsgI or BpmI.
Further, a specific embodiment of the invention concerns a method which is characterized in that for cuts (i) and (ii) the same restriction site is selected.
Further, a specific embodiment of the invention concerns a method which is characterized in that as cut (B) enzyme and as cut (A) enzyme different enzymes are used, preferably BsgI or BpmI as cut (B) enzyme and DraIII as cut (A) enzyme (fd or M13 replication origin is cut).
Further, a specific embodiment of the invention concerns a method which is characterized in that in step (h) and facultatively in step (j) M13K07 is used as the M13-type helper phage.
Further, a specific embodiment of the invention concerns a method which is characterized in that in step (g) selection is made for the presence of an antibiotic resistance gene.
Further, a specific embodiment of the invention concerns a method which is characterized in that in step (i) the multiplicity of infection (MOI) is less than or equal to 1.
Further, a specific embodiment of the invention concerns a method wherein the cosmid comprises an fd or M13 bacteriophage origin.
Further, a specific embodiment of the invention concerns a method wherein in step (e) a mol ratio of display vectors to the cosmid vector within the range 3:1 to 15:1 and preferably 3:1 to 10:1 is used.
Further, a specific embodiment of the invention concerns a method wherein in step (e) a vector concentration (comprising display vectors and cosmid vectors) of more than 100 µg DNA/ml is used.
Another embodiment of the invention concerns a method for the de novo production of large

- phage-display libraries or
- phagemid-display libraries,

comprising DNA sequences aa defined before, and subjectable to recombination according to a procedure as defined before, wherein recombination takes place within a DNA sequence as defined before, wherein

a) a display vector, consisting of an M13 phage or M13-like phage or consisting of a phagemid-display vector comprising a bacteriophage replication origin, facultatively a gene for a selectable marker, preferably an antibiotic resistance, a lambda bacteriophage cos-site and a "stuffer"-sequence (**Figure 5** upper right), containing two binding sites for a type IIs restriction enzyme different from any of the enzymes as defined before (cut (B) and cut (A)), wherein said two sites are oriented in divergent orientation and where the cohesive ends generated on cleavage are non-symmetrical and differ from one another at the two sites, and

b) a PCR-generated fragment comprising part of one of the sequences as defined before, including a (the) hypervariable sequence(s), preferably $X_{n+1}..X_{n+a}Z_{n+a+1}Z_{n+a+2}X_{n+a+3}..X_{n+a+b}$ according to the invention, bracketted by the same type IIs restriction enzyme binding sites defined in (a), but in this case both oriented inwards towards the hypervariable sequence (**Figure 5** left side) and where on cleavage by this restriction enzyme two non-symmetrical, single strand ends different from one another are generated, where the first end (a' in **Fig. 5**) is complementary to one of the ends (a in **Fig. 5**) generated on the large vector fragment in (a) and the second end (b' in **Fig. 5**) is complementary to the other end (b in **Fig. 5**) generated on the large vector fragment in (a),

c) the two cleavage reaction systems (a) and (b) still containing the active type IIs restriction enzyme are mixed together in approximately equimolar proportions and subjected to ligation in the presence of DNA ligase; fragments containing the restriction enzyme binding sites are constantly removed ("stuffer" fragment and outer end of the PCR product) whereas
the other two components, namely the large vector fragment and the insert sequence (central fragment from the PCR reaction) are driven to form

A) a concatameric hybrid if the ligation is carried out at > 100 μg DNA/ml (**Figure 5**), or
B) a circular hybrid if the ligation is carried out at < or = 40 μg DNA/ml,

d1) in the case of protocol A) the DNA is packaged into lambda particles and transduced into an Escherichia coli host,

d2) in the case of protocol B) the DNA is transformed in an Escherichia coli host,

e) if wanted, selection is made for a gene present in the ligated display vectors,

f) the transduced display vectors in the Escherichia coli host are

- either in the case of a phage-display vector spontaneously packaged in M13 or M13-like phage coats
- or in the case of phagemid-display vectors packaged by infecting the Escherichia coli host with an M13-type helper bacteriophage (superinfection),

(g) the packaged display vectors are passaged in a fresh Escherichia coli host and phage-display or phagemid-display libraries are formed and, if wanted

(h) the passaged display vectors are

- either in the case of a phage-display vector spontaneously packaged in an M13 or M13-like phage coats
- or in the case of a phagemid vector packaged by infecting the fresh Escherichia coli host with M13-type helper phages (superinfection) and

phage-display or phagemid-display libraries are formed.

A specific embodiment of the invention concerns a method which is characterized in that in steps (a) to (b), as type IIs restriction enzyme, preferably BpiI, BsgI or BpmI is selected.
Further, a specific embodiment of the invention concerns a method which is characterized in that in step (f) and facultatively in step (h) M13KO7 is used as the M13-type helper phage.
Further, a specific embodiment of the invention concerns a method which is characterized in that in step (e) selection is made for the presence of an antibiotic resistance gene.

Further, a specific embodiment of the invention concerns a method which is characterized in that in step (g) the multiplicity of infection (MOI) is less than or equal to 1.

Another embodiment of the invention concerns a phage-display library or a phagemid-display library in the form of packaged particles obtainable according to any of the methods as described before.

Another embodiment of the invention concerns a phage-display library or a phagemid-display library in the form of display vectors comprised by Escherichia coli population(s) **obtainable** according to any of the methods a s described before.

Another embodiment of the invention concerns a phage-display libraries or phagemid libraries which are characterized by a gene (genes) as defined before and **obtainable** according to the invention, wherein the term "large" as used before is defined as in excess of $10^6$ variant clones, preferentially $10^8$ to $10^{11}$ variant clones.

Finally, another embodiment of the invention concerns a protein or peptide comprising a peptide sequence encoded by a DNA sequence as defined before and obtainable by affinity selection procedures on a defined target by means of a from libraries as defined before.

## Detailed Description

The invention pertains to a novel combination of recombinant DNA technologies to produce large hypervariable gene banks for the selection of novel ligands of pharmaceutical, diagnostic, biotechnological, veterinary, agricultural and biomedical importance with an efficiency higher than was hitherto attainable.

The size of the hypervariable gene bank is *presently considered* the most essential factor limiting the usefullness of the methodology for such purposes, since, as an empirical method, it depends on the *diversity (number of different variants)* initially generated in the bank ( hypervariable gene library). In contrast to this traditional opinion we consider that, when a highly efficient method is developed, as presented here, to generate a large proportion of the possible combinations of mutated segments of the variants from a preselected subpopulation, a population enriched for the desired structural elements will be generated which would only have been represented in a population approaching $N^x$ where N is the size of the original population and x is the number of segments to be recombined.

The first part of the invention pertains to novel sequences which allow recombination within hypervariable DNA sequences encoding regions (domains) variable peptides or proteins displayed in combinatorial phage/phagemid display libraries using type IIs restriction endonucleases both (a) to introduce a cut at the site of recombination and (b) to generate oriented substrates for a ligation reaction, where the ligation products are then recloned at high efficiency after *in vitro* packaging in a lambda packaging mix. The entire protocol yields efficiencies (clones per input DNA) in excess of any described technology (>$10^8$ *clones per microgram ligated DNA).*

Combinations of (vector)sequences and protocols are claimed for both the production of the initial librairies and for recombinational procedures to generate increased diversity within the library or a selected subpopulation at any time. In particular such sequences and procedures are claimed for the generation and use of phage/phagemid-display combinatorial libraries.

The inventors recognise that the main factor thereby determing the efficient generation of further variation is the efficient production of combinatorial libraries from the initial libraries, via reassortment of smaller elements (specific peptide sequences within the hypervariable region, and/or reassortment of structural domains ) which contribute to the properties selected for. The invention presents such a method, which has the unique property that the recombination site may be within the hypervariable region whereby no restriction is imposed on the sequence within the hypervariable region involved. Alternatively the method can be used to reassort domains of proteins or subunits of heteromeric proteins (proteins composed of two or more different variant polypeptide chains), each of which can contain hypervariable regions, without resorting to recloning isolated DNA fragments or generating new libraries containing new synthetic oligonucleotides. It is noted that this method thus offers a saving in both time and materials when optimizing a structure for a predetermined property on the basis of a preselected clone population (subpopulation) and in view of the geometrical increase in possible variability offered may represent a qualitatively novel feature in that some rare structures may be obtainable only by the novel strategy described.

The method, we designate **cosmix-plexing**[7], is based on the design of the cloning vectors, the inserts used and a combination of special recombinant DNA protocols, which in particular use i) cleavage of the phage/phagemid DNA with type IIs restriction enzymes, ii) subsequent ligation to concatamers which are iii) packaged in vitro with a lambda packaging system for iv) efficient transduction into *E.coli* strains, where they are then v) repackaged *in vivo* in filamentous phage coats. The use of **cosmix-plexing**[7] , so defined, on a heterogeneous phage/phagemid population generates an enormous increase in novel variants at any time during further experimentation, e.g. after any enrichment step for structures having the predetermined property or properties.

In particular subpopulations which are enriched from the original library for a specific property will be enriched for a consensus motif ( a degenerate set of related sequences within the varied region(s) which all exhibit the required property to some extent) which may (probably will) include the optimal sequence in terms of the required property. Reassortment of these regions or portions of a single hypervariable sequence by **cosmix-plexing7** will increase the probability of obtaining the optimal sequence. The subpopulations may be isolated by differential affinity-based selection on a defined target, or enrichment procedures based on other desired selectable properties (example 1: substrate properties such as phosphorylation by a particular protein kinase enriched by binding on antibodies which recognise the modified ( in this case phosphorylated) substrate; or example 2: cleavage of the variant sequence by an endoprotease, using selective release of the phage or phagemid previously bound via an interaction between a terminal protein structure(anchor) and its ligand immobilised to, or later trapped on, a surface).

The invention further covers the generation of extension libraries in which *e.g.* a "project-specific cassette" is inserted at the recombination site within the gene bank. Optimisation of ligands can then occur by the generation of further combinatorial libraries from selected clones in which the adjacent regions may be efficiently "shuffled", either singly or both at a time. As far as we are aware no other system provides this "cassette" insertion/exchange" feature.

**Figure legends:**

**Figure 1. Diagrammatic representation of the steps involved in creating recombination within the hypervariable regions of cosmix-plexing7 libraries.**

Double-stranded phagemid from a number of clones (which may be a cosmid itself) and cosmid DNA(if the phagemid is not a cosmid) are cleaved with a type IIs restriction enzyme (cleavage sites indicated by a small bar) within the hypervariable region and ligated together at high DNA concentration so that long concatemers of the DNA molecules are formed, which are all oriented in the same direction, e.g with respect to the M13 packaging origins, i.e. no palindromic regions are formed. The vectors contain one or more restriction site(s) for the type IIs restriction enzyme such that no cohesive ends are formed which on ligation could form palindromic (i.e. head-to-head or tail-to-tail) structures. When the cohesive ends produced on cleavage by the restriction enzyme are themselves non-palindromic and unique to each restriction site within each plasmid/phagemid, only ring closure and the formation of concatemers can be formed. At higher DNA concentrations (i.e. over 200 µg/ml) concatemer formation will be preferred. A more detailed presentation of the molecular structures formed is given in Figures 2 and 3. The ligation product is added to an *in vitro* lambda packaging extract where the DNA is packaged into a lambda bacteriophage coat as a linear DNA of 37 to 50 kb cleaved at a lambda cos-site. In the following step, referred to as transduction, these particles carrying the cosmid-phagemid hybrid DNA are added to Escherichia coli cells (shown as large ellipses in the diagram) into which the DNA injects itself. In the cell it is circularized by closure of the cleaved cos-site using the endogenous DNA ligase. It is then propagated as a large cosmid-phagemid hybrid, replicating from the plasmid DNA replication origin(s). M13-type helper phage (e.g. M13K07) is added to these cells in the step referred to as superinfection. On entry of the helper phage single strand replication is initiated from the M13 replication origins present in the individual copies of the phagemid contained in the concatemer. During this process the phage are also packaged into M13 coats, and secreted into the medium. The phagemid can be harvested from the supernatant of the culture. A second passage, i.e. transduction into an E.coli host and repackaging by superinfection with helper phage is necessary before these phagemid are used in a selection procedure in order to ensure that a particular variant protein is presented only on the particle carrying the gene for that particular variant protein. It is noted that this is a highly efficient process in which a yield of more than $10^8$ different phagemid can be produced pro microgram of ligated input DNA.

**Figure 2.**

The diagram illustrates the DNA structures formed when the cosmix-plexing7 protocol is carried out as shown in Figure 1. Different variants are designated by different patterns for the whole plasmid. Initially double-stranded DNA is cleaved with a type IIs restriction enzyme A. The ligation product is illustrated as a concatemer in which each phagemid is oriented in the same orientation. The products of 37 to 50kb introduced after in vitro lambda packing and introduction into the E.coli cells (shaded ellipses) are shown, whereby, for example 8 to 10 copies of a 4.5kb phagemid may be present per cell. On repackaging the same phagemid are obtained as were present before cleavage and ligation. The protocol as shown here in which the M13-packaging/replication site and the restriction site for enzyme A are identical, is simply an efficient method of amplification when starting with double stranded DNA.

**Figure 3.**

The diagram illustrates a variant of the protocol illustrated in Figures 1 and 2 in which recombination is achieved between different phagemid variants. The cross-over point for the recombination is the cleavage site for the type IIs restriction enzyme B ( shown as a hollow arrow) cleaving preferentially within a hypervariable region or between two different variable regions (see also Figure 4, where additional cleavage sites within other variable regions may be recombined simultaneously). Again, as mentioned in the Figure 1 legend, each phagemid may be a cosmid itself, in which case the addition of another cosmid is unnecessary. In this example cleavage with the restriction enzyme A is optional. Although Figures 2 and 3 are almost identical it should be noted that the products of the scheme in Figure 3 are all recombined, i.e. hybrids of the two sides of different variants. Repassaging is needed before use in the recombined library for selection experiments for the same reasons discussed in the previous two Figures.

**Figure 4. Cosmix-plexing7 strategies.**

The left part of the figure shows the hypervariable DNA sequences encoding the variable portion of the peptide or protein presented on the phage/phagemid. The four bars designated 'N variants' show that there are different sequences on either side of the type IIs restriction cleavage site. Phagemid DNA from the variant clones can be cleaved with the type IIs restriction enzyme and religated to yield the indicated number of recombinant clones, within the limits of the cloning efficiency. If one starts with a subpopulation of preenriched variants from the primary library (say $4x10^4$ clones) then one-sixteenth of all possible recombinants ($10^8$) can be obtained.

The construction of "extension libraries" is shown below the dotted line. In this case a project-specific-cassette containing a biased codon distribution encoding some sequence elements previously defined as advantageous for binding to the target is inserted into the hypervariable sequence at the type IIs restriction cleavage site. The large library thus generated encodes a protein containing three segments (domains B, A and C), whereby the central domain A is encoded by the project-specific cassette, and is bordered by the hypervariable domains B and C.

The formuli for the numbers of variants obtained are made for the protocol in which four separate libraries are constructed.

The right side of the figure illustrates how the variant protein might bind to a target protein. The variants selected from the extension library are expected to have a larger surface of interaction and thus to exhibit stronger and /or more specific binding to the defined target. The target may be a cell, a (partially) purified protein or peptide e. g. enzyme, antibody, hormone or lymphokine, cell receptor or in fact any defined surface or particle suspension, possible coated with one of the aforementioned targets, which is amenable to physical separation, i.e. the wall of a receptacle (tube, tubing, flask, microtiter plate, a planar surface), or a particle ( e.g. beads, magnetic beads, or droplets in a two-phase liquid system).

**Figure 5: Driven directed cloning (DDC)**

This figure illustrates an example of a cloning protocol which has excellent properties for the highly efficient construction of hypervariable libraries and extension libraries, which can be used with the cosmix-plexing7 method. The left side of the figure shows the preparation of the hypervariable cassette to be inserted into the cosmid-phagemid double-stranded vector. The cosmid-phagemid vector containing a "stuffer fragment" is shown on the right. Both the PCR-product containing the hypervariable sequence, shown as a line of asterisks, and the vector containing the "stuffer" are cleaved with the same type IIs restriction enzyme(s). It is noted that the recognition sites for this(these) enzyme (s) are oriented in opposite directions, i.e. outwards from the stuffer in the case of the vector, and inwards in the case of the PCR-product. After cleavage neither the hypervariable cassette to be inserted nor the vector contain any of the original type IIs restriction enzyme recognition sites. The vectors and insert are, however designed to have non-palindromic cohesive ends at their termini, generated by the restriction enzyme cleavage, so that a ligation of insert and vector leads to an oriented insertion of the hypervariable region. In addition, the vector cannot undergo ring closure in the absence of the insert cassette nor can the insert fragments ligate to one another. Since the ligation is carried out at high DNA concentration and in the continued presence of the restriction enzyme any ligation product resembling the in initial uncleaved or partially cleaved vector or PCR-product will be immediately recleaved. This combination of oriented non-palindromic cohesive ends and recleavage of unwanted ligation products, drives, especially at high DNA concentration, where the formation of ring closure of a vector-insert-hybrid is at a disadvantage, the formation of oriented double-stranded concatemers of the structure required for highly efficient cosmid packaging. The primary cosmix-plexing library is formed finally by transducing the packaged cosmid-phagemid hybrids into an E.coli host which contains, or is superinfected with, an M13-like helper phage. The phagemid are repassaged in a second M13 phage-packaging step before use in selection so that individual phage clones are derived from singly infected cells. This is necessary in order that each phagemid particle carries the variant encoded in its genome.

This is not the situation in the first packaging step in which the E.coli host contains a concatemer of some eight different variant phagemid.

Recombination can be acheived within the hypervariable region of the gene encoding the protein or peptide presented on the phagemid according to the scheme illustrated in Figure 1. With extension libraries, either the left (5') or right (3') extension, or both, can be reassorted by cleaving with a type IIs restriction enzyme recognizing a site bordering either left end, the right end (opposite orientation), or both ends respectively, as described for the sequences $B_1$-$B_n$ and $Q_{n+a+1}$...$Q_{n+a+i}$ in Claim 3.

The use of hypervariable sequences in the description of the invention implies in general that we try to use set of oligonucleotides in which "randomized sequences" encode amino-acids at ratios near to that normally found in natural proteins, whereby the frequency of stop-codons is reduced. We are aware that for certain applications biased subsets may be preferable in the construction of dedicated sublibraries.

Example 1

Cosmixplexing using the four-tube-method (according to claims 1-10)

1a) Library generation

Oligonucleotide Sequences:

NONA-CA:

5′ TCGG**GGTACC**TGGAGCA(XNN)$_4$KKN(XNN)$_4$GCTGCACGG**GAGCTC**GCC 3′
*KpnI* *SacI*

NONA-CT:
5′ TCGG**GGTACC**TGGAGCA(XNN)$_4$RRN(XNN)$_4$GCTGCACGG**GAGCTC**GCC 3′

NONA-GA:
5′ TCGG**GGTACC**TGGAGCA(XNN)$_4$YYN(XNN)$_4$GCTGCACGG**GAGCTC**GCC 3′

NONA-GT:
5′ TCGG**GGTACC**TGGAGCA(XNN)$_4$MMN(XNN)$_4$GCTGCACGG**GAGCTC**GCC 3′

where X means: A, C and G; N: A, C, G and T; K: G and T; R:G and A; Y: C and T; M: C and A.

NONA PCR-L:
5′ GGC**GAGCTC**CCGTGCAGC 3′

NONA PCR-R:
5′ TCGG**GGTACC**TGGAGCA 3′

*Kpn*I (GGTACC) and *Sac*I (GAGCTC) restriction enzyme recognition sites are marked in bold type.

important vector DNA-Sequences:

pROCOS4/7:

*Eco47*III

*Sac*I *Bsg*I *Kpn*I |*pIII*→

5′ GGC**GAGCTC**CC**GTGCAGCGCTCCAGGTACC**CCGATATCAGAGCTGAA 3′

*Bpm*I

pROCOS4/7-Stuffer1:

*Eco47*III *Eco47*III

*Sac*I *Bsg*I *Kpn*I

|*pIII*→

5′ GGC**GAGCTC**CC**GTGCAGC*GCT*...** ...***AGC*GCTCCAGGTACC**CCGATATCAGAGCTGAA

3′ *Bpm I*

⇑

952 bp *Eco47*III fragment

of plasmid pBR322

*Kpn*I (GGTACC), *Sac*I (GAGCTC), *Bsg*I (GTGCAG), *Eco*47III (AGCGCT) and *Bpm*I (CTGGAG) restriction enzyme recognition sites are marked in bold type. The first codon of the mature pill protein (GAA) is indicated.

For the generation of double-stranded DNA inserts the single-stranded hypervariable DNA oligos NONA-CA, NONA-CT, NONA-GA and NONA-GT are amplified using the single stranded DNA oligos NONA PCR-L and NONA PCR-R as PCR-primers according to the following protocol:

Remark: the four hypervariable DNA-oligos have to be kept strictly separated!

PCR-Amplification of DNA Oligos

| PCR-buffer (10X): | |
|---|---|
| KCl | 500 mM |
| Tris-HCl (pH 9.0) | 100 mM |
| Triton X-100 | 1 % |

| Taq DNA polymerase (Promega) in storage buffer A: | |
|---|---|
| glycerol | 50 % |
| Tris-HCl (pH 8.0) | 50 mM |
| NaCl | 100 mM |
| EDTA | 0.1 mM |
| DTT | 1 mM |
| Triton X-100 | 1 % |

| TE-buffer (1X) | | |
|---|---|---|
| Tris-HCl | (pH 8.0) | 10 mM |
| EDTA | 0.1 mM | |

1. Transfer 2 µl of a 10 pmol/µl solution of the hypervariable oligos NONA-CA, -CT, -GA and -GT in a 0.2 ml PCR reaction tube (4 tubes).
2. Mix the following in one Eppendorf reaction tube:

| | |
|---|---|
| $ddH_2O$ | 276.75 µl |
| PCR-buffer (10X) | 45.0 µl |
| NONA PCR-L (100 pmol/µl) | 9.0 µl |
| NONA PCR-R (100 pmol/µl) | 9.0 µl |
| dNTPs (10 mM each) | 9.0 µl |
| Taq DNA polymerase (5 U/µl) | 2.25 µl |

3. Transfer 78 µl of this mixture to each of the PCR tubes containing the hypervariable oligos (step 1).
4. Mix 45 µl $MgCl_2$ (25 mM) and 45 µl $ddH_2O$ in an Eppendorf reaction tube.
5. Preheat a PCR thermocycler to 94 °C (if possible use a heated lid).
6. Transfer 20 µl of the $MgCl_2$ solution (step 4) into each of the PCR tubes (step 3).
7. Put the tubes directly into the thermocycler (simplified hot-start) and run the following program:

    1. 94 °C 30 sec
    2. 94 °C 10 sec
    3. 52 °C 10 sec
    4. repeat 9 times step 2 and 3
    5. hold at 4 °C

8. Take an aliquot of 5 µl to run a 4.5 % agarose gel.
9. Add 200 µl $ddH_2O$ to each tube, extract with phenol, precipitate with ethanol and resuspend the DNA in 120 µl TE-buffer.

For cloning the amplified oligo-DNA are cut with *Kpn*I and *Sac*I. Also the vector-DNA has to be cut with both enzymes. As vector-DNA pROCOS4/7 or a derivative thereof named pROCOS4/7-Stuffer1 which contains a DNA-Stuffer fragment for easier control of the double digest reaction can be used without any consequences regarding the final cloning results. Digestions are done according to the following protocols:

| buffer B + TX-100 (1X) | |
|---|---|
| Tris-HCl (pH 7.5) | 10 mM |
| MgCl2 | 10 mM |
| BSA | 0.1 mg/ml |
| Triton X-100 | 0.02 % |

| buffer A (1X) | |
|---|---|
| Tris-acetate (pH 7.9) | 33 mM |
| Mg-acetate | 10 mM |
| K-acetate | 66 mM |
| Dithiothreitol | 0.5 mM |

Vector DNA Digestion

1. For the restriction digestion of the vector DNA with *Kpn*I set up the following mixture:

| | |
|---|---|
| pROCOS4/7-Stuffer1 | X µl (200 µg) |
| buffer B + TX-100 (10X) | 150 µl |
| BSA (10 mg/ml = 100X) | 15 µl |

(continued)

| | |
|---|---|
| *Kpn*I | X µl (400 U) |
| $ddH_2O$ | to 1500 µl |

incubate at 37 °C for 3 hr and stop the reaction by incubating at 65°C for 20 min.
2. Take an aliquot of 3 µl and run a 1 % agarose gel with uncleaved DNA as a control.
3. Extract with phenol, precipitate with ethanol and resuspend the DNA in 820 µl TE-buffer.
4. Store a 20 µl aliquot of the digested DNA at - 20 °C and mix the following for the digestion with *Sac*I:

| | |
|---|---|
| pROCOS4/7-Stuffer/*Kpn*I | 800 µl |
| buffer A (10X) | 100 µl |
| *Sac*I | X µl (400 U) |
| $ddH_2O$ | 1000 µl |

incubate at 37 °C for 3 hr.
5. Take an aliquot of 3 µl and run a 1 % agarose gel using uncleaved and single-cut DNA as a control.
6. Extract with phenol, precipitate with ethanol and resuspend the DNA in 550 µl TE-buffer.

Oligo DNA Digestion

1. For the digestion of double-stranded (ds) oligo DNA with *Kpn*I set up the following four mixtures:

| | |
|---|---|
| NONA-CA, -CT, -GA or -GT dsDNA | 100 µl |
| buffer B + TX-100 (10X) | 50 µl |
| BSA (10 mg/ml = 100X) | 5 µl |
| *Kpn*I | X µl (400 U) |
| $ddH_2O$ | to 500 µl |

incubate at 37 °C for 5 hr.
NOTE: Don't heat up the oligo DNA.
2. Take an aliquot of 5 µl and run an 4.5 % agarose gel with uncleaved DNA as a control.
3. Extract with phenol, precipitate with ethanol and resuspend the DNA in 110 µl TE-buffer.
4. Store an 10 µl aliquot of the digested DNA at - 20 °C and set up the following four mixtures for the digestion with *Sac*I:

| | |
|---|---|
| NONA-CA, -CT, -GA or -GT/*Kpn*I | 100 µl |
| buffer A (10X) | 50 µl |
| *Sac*I | X µl (400 U) |
| $ddH_2O$ | to 500 µl |

incubate at 37 °C for 5 hr.
5. Take an aliquot of 5 µl and run an 4.5 % agarose gel using uncleaved and single-cut DNA as a control.
6. Extract with phenol, precipitate with ethanol and resuspend the DNA in 55 µl TE-buffer.

The vector-DNA fragment may be purified using the following protocol:

Purification of Vector DNA Fragments by Gel Extraction

1. To separate the pROCOS4/7 vector DNA fragment from the stuffer fragment prepare a horizontal 1 % agarose gel using a one-tooth combs.
2. Mix the DNA with 1/10 vol gel loading buffer, load onto the gel and electrophorese at 100 V until both fragments are clearly separated.
3. Put the gel on the UV transilluminator and excise the 5.5 kb pROCOS4/7 vector DNA fragment.
4. Extract the agarose slice using the JETsorb gel extraction kit (Genomed GmbH, Germany).

Vector- and insert DNA fragments are ligated and transformed according to the following protocols:

Ligation of DNA Fragments

Check the integrity of vector and insert DNA fragments by agarose gel electrophoresis (1 % and 4.5 % respectively). The concentration of the insert DNA may be estimated by comparing its ethidium bromide staining with standards of known quantity like assembled oligonucleotides. To determine the vector DNA concentration determine the absorbance at 260/280 nm.

| T4 DNA ligase buffer (1X): | |
|---|---|
| Tris-HCl (pH 7.5) | 50 mM |
| $MgCl_2$ | 10 mM |
| Dithiotreitol | 10 mM |
| ATP | 1 mM |
| BSA | 25 μg/ml |

Test Ligation

1. To determine the appropriate ratio of insert to vector DNA a series of test ligations may be performed. For this assemble ligation reactions composed of:

| | |
|---|---|
| vector DNA fragment | X μl (0.5 μg) |
| T4 DNA ligase buffer (10X) | 1 μl |
| $ddH_2O$ | to 9 μl |

2. Prepare three twofold dilutions of the insert DNA's in $ddH_2O$ and add 1 μl of undiluted DNA as well as 1 μl of each dilution to one of the ligations reactions.
NOTE: The aim of this is to create vector to insert DNA (V/I) ratios of 1:5 to 2:1.
3. Add 1 unit T4 DNA ligase to each reaction and incubate overnight at 15 °C.
NOTE: As a control one reaction without insert DNA and one without ligase should be included.
4. Add 1 vol $ddH_2O$ to each reaction and incubate at 65 °C for 10 min.
5. Precipitate the DNA with ethanol and resuspend it in 10 μl TE buffer.
6. Transform electrocompetent *E. coli* JM110λ cells with the content of each tube and plate dilutions on ampicillin containing LB agar plates.

Large-Scale Ligation

1. To create the libraries set up four of the following mixtures:

| | |
|---|---|
| vector DNA fragment | X μl (1/4 of the total prep.) |
| insert DNA | X μl (to create the optimal V/I-ratio) |
| T4 DNA ligase buffer (10X) | X μl (1/10 of the final vol) |
| T4 DNA ligase | X μl (2 U/μg DNA) |
| $ddH_2O$ | to create a DNA conc. of 0.05 μg/μl |

incubate overnight at 15 °C.

2. Extract with phenol, precipitate with ethanol and resuspend each of the ligation mixtures in sufficient TE-buffer to adjust the DNA concentration to 0.1-0.2µg/µl.

Preparation of Competent Cells

1. Inoculate 20 ml of LB medium with a single colony of *E. coli* JM110λ and incubate at 37°C and 180 rpm overnight.
2. Next day inoculate 2x1 liter of LB medium (2 x 21 Erlenmeyer flask) at 1 % with the overnight grown culture and incubate again at same conditions until an optical density of $OD_{600}$ = 0.6 has been reached.
3. Transfer 250 ml aliquots of the culture into centrifuge tubes (GS3), chill the cells on ice and centrifuge for 15 min at 8000 rpm and 4 °C (Sorvall RC5C centrifuge; GS3 rotor). Decant the supernatant.
4. Resuspend each pellet in 250 ml of ice-cold $ddH_2O$, centrifuge again (step 3) and decant the supernatant.
5. Resuspend each pellet in 125 ml of ice-cold $ddH_2O$, collect each of two aliquots in one tube, centrifuge again (step 3) and decant the supernatant.
6. Resuspend each pellet in 10 ml of ice-cold sterile glycerol (10 %), collect all of the aliquots in one GSA centrifuge tube, centrifuge for 15 min at 8000 rpm and aspirate the supernatant.
7. Resuspend the bacterial pellet in 10 ml of ice-cold sterile glycerol (10 %).
8. Fill aliquots of 100 µl in precooled, sterile Eppendorf reaction tubes, freeze immediately in liquid nitrogen and store at -70 °C.

Transformation of *E. coli* Cells by Electroporation

1. Place freezed aliquots of competent *E. coli* cells on ice and let them thaw.
2. To each aliquot add up to 2 µg DNA in less than 10 µl and incubate on ice for 1 minute.
3. Fill the suspension in a prechilled electroporation cuvette (0.2 cm pathlength), place the cuvette in the electroporation sled and give a pulse at a voltage of 2.5 kV, a capacity of 25 µF and a resistence of 200 Ω (Gene Pulser and Puls Controller, Bio-Rad).
4. Immediately add 1 ml of LB medium (supplemented with 20 mM Glucose), mix and transfer the suspension in an Eppendorf reaction tube.
5. Incubate for 1 hour at 37 °C and plate on LB agar plates containing ampicillin (100 µg/ ml). Incubate overnight at 37 °C.
NOTE: To determine the size of the libraries also plate dilutions of the transformed cells.
6. To create library stocks resuspend the cells in LB/ampicillin medium, mix with 1 vol of sterile 87% glycerol and store at -70 °C.

1b) Recombination

For recombination within the hypervariable Sequences according to the four tube cosmixplexing method the libraries can be preselected. For this purpose the *E. coli* cells containing the phagemid libraries are superinfected with M13K07 helperphages, progeny phages presenting fusionproteins are harvested and used for the first round of a panning according to standard methods e.g.:

Preparation of M13K07 Phage Stocks

PEG/NaCl-solution:
(16.7 %/3.3 M)
100 g PEG 8000
116.9 g NaCl
475 ml $H_2O$
PBS-buffer (1X):
8.0 g NaCl
0.2 g KCl
1.43 g $Na_2HPO_4$ +2 $H_2O$
0.2 g $KH_2PO_4$
$H_2O$ ad 1 l

pH 6.8 - 7

1. Use a disposable pasteur pipette to pick a single, well separated M13K07 plaque from a *E. coli* WK6 lawn grown overnight on a LB/kanamycin (Km) plate, inoculate 20 ml of LB(2X)/Km medium (100 ml Erlenmeyer flask) with this agar slice and incubate overday at 37 °C on a shaker at 180 rpm.
2. Inoculate 2 x 500 ml LB(2X)/Km medium (in 2 l Erlenmeyer flasks) with 10 ml preculture and incubate overnight (37 °C, 180 rpm).
3. Next day centrifuge four 250 ml aliquots for 15 minutes at 8,000 rpm and 4 °C (Sorvall RC5C centrifuge; GS3 rotor). Transfer the supernatant into centrifuge bottles, centrifuge and transfer the supernatant again into fresh centrifuge bottles.
4. Add 0.15 vol. of PEG/NaCl solution, mix and incubate on ice for at least 2 hours.
5. Centrifuge for 60 min at 8000 rpm (GS3 rotor), decant the supernatant, centrifuge for some sec at up to 4000 rpm and remove last traces of the supernatant using a pipette.
6. Resuspend each PEG-pellet in 2.5 ml PBS solution and collect the resuspended phages in one SS34 centrifuge bottle. To clear the suspension centrifuge again for 10 min at 12000 rpm (SS34 rotor). Recover the supernatant (pipette), add $NaN_3$ to a final concentration of 0.02 % and store the phages at 4 °C.

Packaging of Phagemids (keep each library separate!)

1. Inoculate 100 ml of LB/Amp medium (1 l Erlenmeyer flask) with 1 ml of *E. coli* JM110λ cells containing phagemids (from overnight culture or resuspended cells) and incubate at 37 °C and 180 rpm until $OD_{600}$ = 0.5 (~ 2.5 h).
2. Add 500 µl M13K07 stock solution ($10^{11}$ - $10^{12}$ cfu/ml), incubate at 37 °C for 15 min and continue shaking at 37 °C and 180 rpm overnight.
3. Next day centrifuge for 10 min at 8000 rpm (GSA rotor), decant the supernatant into a fresh bottle and repeat the centrifugation step.
4. Add 0.15 Vol of PEG/NaCl solution and incubate on ice for at least two hours.
5. Centrifuge for 60 min at 10000 rpm (GSA rotor), decant the supernatant and repeat the centrifugation and remove the supernatant completely.
6. Dissolve the pellet in 1 ml of PBS buffer and transfer the solution into an Eppendorf reaction tube. Centrifuge for 10 min at 13000 rpm (batch centrifuge), recover the cleared solution and add $NaN_3$ (final concentration of 0.02 %). Store at 4 °C.

Panning Procedure (keep each library separate!)

T-PBS solution:
PBS-buffer containing 0.5 % Tween 20
Blocking solution:
PBS-buffer containing 2 % skim milk powder
Elution-buffer:
glycine (0.1 M; pH 2.2)

1. Coating of Microtiter Plates:

Fill 100 µl of ligand solution (100 µg/ml PBS) into the wells of a 96-well microtiter plate (Nunc maxisorb) and incubate overnight at 4 °C or at least 2 hours at room temperature. Shake out the wells, slap the plate onto a paper towel and wash the wells once with T-PBS solution (ELISA plate washer or manually).

2. Blocking:

Fill the wells with 400 µl of blocking solution and incubate at room temperature for ~ 1 hour. Shake out the wells, slap the plate onto a paper towel and wash the wells once with T-PBS.

3. Binding:

Fill the coated and one uncoated well (as a control) with 100 µl of phage preparations diluted 1:1 with skim milk powder (usually ~ $10^{10}$ - $10^{11}$ phages/well) and incubate at room temperature for 1 to 3 hours.

4. Washing:

Remove the solutions using a pipette and slap the plate onto a paper towel.
In the first round of panning wash the wells once with T-PBS, incubate for 10 min with 400 µl blocking solution, wash again with T-PBS and finally two times with water. During all further rounds repeat the T-PBS washing steps three times. All washing steps can be carried out manually using a pipette or with an ELISA plate washer.

5. Elution:

Slap out the plate and fill the wells with 100 µl of elution-buffer, incubate at room temperature for 15 min and transfer the solution into an Eppendorf reaction tube containing 6 µl Tris (2 M) .

6. Determine the titer of eluted phages as described under 3.1.3.

Reinfection of *E. coli* Cells (keep each library separate!)

1. Mix the eluted phages and 10 ml of *E. coli* JM110λ log-phase cells and incubate for 30 min at 37 °C..
2. Collect the cells by centrifugation (5 min, 8000 rpm, SS34 rotor) and resuspend the pellet in 400 µl of LB/Amp medium.
3. Plate each suspension on one LB/Amp agar plate (Ø 14.5 cm) and incubate overnight at 37 °C.

After one round of panning populations of about $10^5$ individual clones enriched towards binding one's are expected. For recombination the phagemid DNA has to isolated according to standard protocols, e.g.:

Preparation of Phagemid-DNA from Reinfected Cells

1. Resuspend reinfected *E. coli* cells in 20 ml of LB/Amp medium and use 200 µl for the inoculation of 3 ml LB/Amp medium.
2. Incubate at 180 rpm and 37 °C for 1 hour.
3. Prepare the DNA using Jetquick Plasmid Miniprep Spin Kits (Genomed GmbH, Germany) according to the instructions of the supplier.

Using this method up to 30 µg of DNA can be isolated. For pROCOS4/7 based libraries the phagemid size is 4.3 kb corresponding to a molecular weight of 2.9x $10^6$ g/mol or round about 2 x $10^{11}$ phagemid molecules/µg DNA. Therefore 10 µg of recombined DNA contains more molecules than the theoretical number of different variants that can be created from $10^5$ clones $((10^5)^2=10^{10})$.
For recombination the phagemid DNA of each preselected library is cut separately e.g. with *Bpm*I or alternatively with *Bsg*I:

Digestion of the phagemid DNA

| NEB3-buffer (1X) | |
|---|---|
| NaCl | 100 mM |
| Tris-HCl (pH 7.9) | 50 mM |
| $MgCl_2$ | 10 mM |
| Dithiothreitol | 1 mM |

1. Set up the following reaction

| | |
|---|---|
| phagemid-DNA | 10µg |
| *Bpm*I (2u/µl) | 5µl |
| NEB3 (10X) | 4µl |

(continued)

| | |
|---|---|
| BSA (1 mg/ml) | 4µl |
| H2O | up to 40 µl |

incubate at 37 °C for 5 hr.
2. Take an aliquots of 4 µl and run a 1 % agarose gel to check the digestion.
3. Extract with phenol, precipitate with ethanol and resuspend the DNA in TE-buffer.

Digested phagemid-DNA's are religated at high concentration (≥ 0.2 µg/µl) to favour formation of concatemers, packaged into λ phage particles and used for the transfection of *E. coli* cells (according to "Packaging of Bacteriophage λ DNA in vitro; protocol I" p.2.100-2.104, in: Molecular Cloning- a Laboratory Manual, Sambroock et al. (eds.), 2. ed., 1989, Cold Spring Harbour Laboratory Press). Transfected phagemids are separated by packaging reinfection using M13K07 helperphages (see above).

Example 2

Cosmixplexing using the one-tube-method (according to claims 11-17 and 44)

2a) Library generation

Oligonucleotide sequences:

NONACOS-NGG: B*pi*I *Bsg I*

5′ GGCTCTGATG**GAAGACGT↓GCAG**C(NNB)$_4$NGG(NNB)$_4$TG**C↓TCCAG**A**GTCTTC**CTC

↑ B*pm*I ↑ *Bpi*I

CTGTCG 3′

NONACOS-NCT:

5′ GGCTCTGATG**GAAGACGTGCAG**C(NNB)$_4$NCT(NNB)$_4$TG**CTCCAG**A**GTCTTC**CTCC

TGTCG 3′

NONACOS-NAM:

5′ GGCTCTGATG**GAAGACGTGCAG**C(NNB)$_4$NAM(NNB)$_4$TG**CTCCAG**A**GTCTTC**CTC

CTGTCG 3′

NONACOS-NTS:

5′ GGCTCTGATG**GAAGACGTGCAG**C(NNB)$_4$NTS(NNB)$_4$TG**CTCCAG**A**GTCTTC**CTC

CTGTCG 3′

where N means: A, C, G or T; B: C, G or T; M: A or C and S: C, G or T.

NONACOS-PCR-L: *Bpi*I *Bsg*I

5' GGCTCTGATG**GAAGACGTGCAG** 3'

NONACOS-PCR-R:*Bpi*I *Bpm*I

5' CGACAGGAG**GAAGAC**T**CTGGAG** 3'

*Bpi*I (GAAGAC), *Bsg*I (GTGCAG) and *Bpm*I (CTCCAG) restriction enzyme recognition sites are marked in bold type. *Bpi*I cutting sites are marked by arrows.

important vector DNA-sequences of pROCOS5/3:

*Bsg*I *Eco47*III

5' GGCGAGCTCCC**GT↓GCAG**CG**GTCTTCAGCGCT**TGCCGTCTGACCGT

↑ *Bpi*I

*Eco47*III BpiI |*pIII*→

**AGCGCT**G**GAAGAC**G**C↓TCCAG**AGGGTACCCCGATATCAGAGCTGAA 3'

*Bpm*I ↑

*Bpi*I (GAAGAC), *Bsg*I (GTGCAG) *Eco*47III (AGCGCT) and *Bpm*I (CTCCAG) restriction enzyme recognition sites are marked in bold type. *Bpi*I cutting sites are marked by arrows. The first codon of the mature pIII-Protein (GAA) is also indicated.

To create libraries according to the one-tube method the hypervariable oligos NONACOS-NGG, -NCT, -NAM and -NTS are amplified using the PCR-primer NONACOS-R and NONACOS-L as described in example 1, except that the oligo-DNA's don't have to be kept separate.

After this pROCOS5/3-vector-DNA and double stranded (ds) oligo-DNA are digested with *Bpi*I and ligated at the same time according to the following Protocol:

Digestion/Ligation

1. Set up the following mixture:

| | |
|---|---|
| pROCOS5/3 DNA | 200 μg |
| NONACOS-NGG, -NCT, -NAM and -NTS ds DNA | 100 μl |
| *Bpi*I | 200 u |
| buffer G (10x) | 40 μl |
| BSA (10 mg/ml) | 4 μl |
| $H_2O$ | up to 400 μl |

incubate at 37 °C for 2 hr, add 200 units T4 DNA ligase and continue the incubation at 15 to 30 °C over night.

2. Take an aliquot of 3 μl and run an 1 % agarose gel as a control.

This protocol favours the production of concatemers of the desired product, that can be packaged for example in *E. coli* JM110λ cells by λ-packaging according to example 1.

2b) Recombination

For panning and recombination the same methods as described for example 1 can be used, except that one library is used instead of four separate libraries.

**List of reference signs**

Figure 1.

1 Phagemid library *in vitro*
2

1. Type IIs restriction and ligation
2. *In vitro* packaging in a lamda packaging mix
3. Transduction into *E.coli*
   Efficiency ≥ $10^7$/microgram ligated DNA

3 cosmid containing a concatamer of phagemid
4 Superinfection by M13K07
5 ca. 7 to 8 different phagemid are released per E.coli clone Total efficiency ≥ $10^8$/microgram ligated DNA
6 Phagemid particles each presenting different variant proteins

Figure 2.

1 Enzyme A(type IIS) cleavage and ligation to concatamers
2 In vitro lambda packaging + transduction in *E.coli*
3 M13-helper phage superinfection (repackaging of individual phagemid from the concatenate)
4 What one puts in is what one gets out.

Figure 3.

1 M13 replication/packaging origin=type IIs enzyme A cleavage site
2 Enzyme A + B (type IIs) cleavage and ligation to concatamers
3 In vitro lambda packaging + transduction in *E.coli*
4 M13-helper phage superinfection (repackaging of individual phagemid from the concatenate)
5 The left and right of the type IIs cleavage site B are recombined.

Figure 4.

Cosmix-plexing® strategies

1 Reassortment after cleavage and repackaging
2 project-specific cassette
3 Reassortment after cleavage and repackaging
4 target
5 panning selection with a standard cosmix-plexing® library
6 primary selected ligand (domain A)
7 panning selection with a cosmix-plexing® inserting the consensus cassette from the initial library into an extension library

8 secondary optimized ligand selected from an extension library (domains B, A + C; B+C from the extensions)
9 continue optimization?

1. reassort, left and or right extension
2. insert another cassette->reinsert central cassettes in new extension library
3. repeat any steps .....

Figure 5.

Driven directed cloning (DDC) for the construction of primary libraries

1 hypervariable region
2 type IIs restriction enzyme cleavage
3 Ligation in the presence of restriction enzyme
4 "stuffer"
5 Cosmid packaging and phagemid packaging + M13-type helper phage
6 hybrid phagemid

## Claims

**1.** Use of a typells restriction enzyme for recombining variable DNA sequences in an ordered fashion for the generation of a variable non-palindromic cohesive end at the desired recombination site within the variable DNA sequence, wherein the recognition site for the type IIs restriction enzyme is located adjacent to the variable DNA sequence.

**2.** A process for generating DNA sequences with desired properties from a library by selection and recombination, wherein the library comprises hypervariable DNA sequences, comprising at least the following steps:

- selection of library members with desired properties
- optimisation of the isolated subset of hypervariable DNA-sequences by recombining said DNA-sequences of the isolated subset by the use of at least one type IIs restriction enzyme
- wherein the typells restriction enzyme recognises a recognition site which is located adjacent to the hypervariable DNA sequence and cleaves within the hypervariable DNA sequence thus generating a recombination site
- selection of members from the recombined subset of DNA sequences which have the desired properties
- optionally performing further rounds of selection and recombination.

**3.** A process according to claim 1 wherein the type IIs restriction enzyme creates non-palindromic cohesive ends.

**4.** A process according to claim 2 or 3 wherein an oligonucleotide cassette of a bases in length is inserted into the recombination site of the hypervariable sequences thus placing the oligonucleotide cassette inbetween the left and right segment of the hypervariable DNA sequences and thus providing an extension library.

**5.** A process according to one of the above claims wherein the oligonucleotide cassette is a project specific cassette obtained from the initial library by a selection and recombination process according to claim 2.

**6.** A process according to one of the above claims wherein the left and/or right extensions are recombined by the use of at least one typells restriction enzyme which recognises at least one recognition site in the vector which is located adjacent to the extension DNA sequences and cleaves within the extension DNA sequences thus generating a recombination site for reassorting left and/or right extensions.

**7.** A process according to one of the above claims wherein optionally further oligonucleotide cassettes are inserted.

**8.** A process according to one of the above claims wherein the extension library members are optimised by recombination and selection.

**9.** A process according to one of the above claims wherein the DNA sequence selected from an extension library encoding an optimised protein or peptide is reinserted at the recombination site of hypervariable DNA sequences thus creating a new extension library.

**10.** Method for recombining segments of variant DNA sequences present in a vector in an ordered fashion wherein the recombination site is created by the use of at least one type IIs restriction enzyme which recognises a recognition site located adjacent to the variant DNA sequence and which cleaves within the variant DNA sequence thus providing a cohesive end for recombination wherein inverted sequences at the recombination site are avoided within pools of DNA sequences which can recombine with each other wherein the cleaved vectors are ligated thus allowing the formation of new sequence variants.

**11.** Method according to claim 10 wherein upon ligation of the cleaved vectors concatemers are formed wherein all vectors are oriented in the same direction.

**12.** Method according to claim 10 or 11 wherein the vectors are phagemid vectors.

**13.** Method according to one of the above claims 10 to 12 wherein the transformation efficiency of Escherischia coli cells is increased by introducing several phagemid vector sequence at once into the host cell by the use of a at least one cosmid vector.

**14.** Method according to one of the above claims 10 to 13 wherein the cleaved vectors are ligated in the presence of cleaved cosmid vectors having ends which can be ligated to the cleaved vectors thus allowing the creation of at least one fusion product consisting of cleaved vectors and cosmid vectors.

**15.** Method according to one of the above claims 10 to 14 wherein the ligation product is added to an in vitro lambda packaging extract where the DNA is packaged into an lambda bacteriophage coat as a linear DNA of 37 to 50 kb cleaved at a lambda cos-site.

**16.** Method according to one of the above claims 10 to 15 wherein the particles carrying the cosmid/phagemid hybrid DNA are added to the Escherischia coli cell wherein the linear DNA is injected into the cells and circularized by closure of the cleaved cos site using the endogenous DNA ligase.

**17.** Method according to one of the above claims 10 to 16 wherein a M13-type helper phage is added initiating single strand replication from the M13 replication origins present in the individual copies of phagemid contained in the concatemer leading to the resolution and release of phagemid vectors carrying recombined DNA sequences.

**18.** Method for the construction of hypervariable cosmid-phagemid libraries which can be used in a process according to claims 2 and/or 10 wherein hypervariable DNA sequence cassettes are inserted into cosmid-phagemid vectors, wherein

- each vector comprises at least two recognition sites for at least one typeIIs restriction enzyme bracketed around a stuffer sequence at a position where at least one hypervariable cassette is to be inserted, wherein the recognition sites are oriented outwards of the stuffer sequence
- wherein the hypervariable cassettes are part of PCR products, wherein each PCR product comprises at least two recognition sites for at least one typeIIs restriction enzyme wherein the recognition sites are orientated inwards
- wherein the vectors and the PCR-products are cleaved with at least one typeIIs restriction enzyme recognising the recognition sites as defined above
- wherein the vectors and the hypervariable cassettes are designed to have non-palindromic cohesive ends at their termini generated by the typeIIs restriction cleavage,
- wherein the vectors and the hypervariable cassettes are ligated.

**19.** Method according to claim 18 wherein ligation is carried out in presence of the used typeIIs restriction enzyme(s).

**20.** Method according to claim 18 or 19 wherein upon ligation at the non-palindromic cohesive ends oriented concatemers are formed.

**21.** Method according to one of the above claims 18 to 20 wherein the cosmid-phagemid vectors carrying the hypervariable cassette are packaged and transduced into an Escherischia coli host strain.

**22.** Method according to one of the above claims 18 to 21 wherein an M13-like helper phage is either contained or added to the host.

Figure 1.

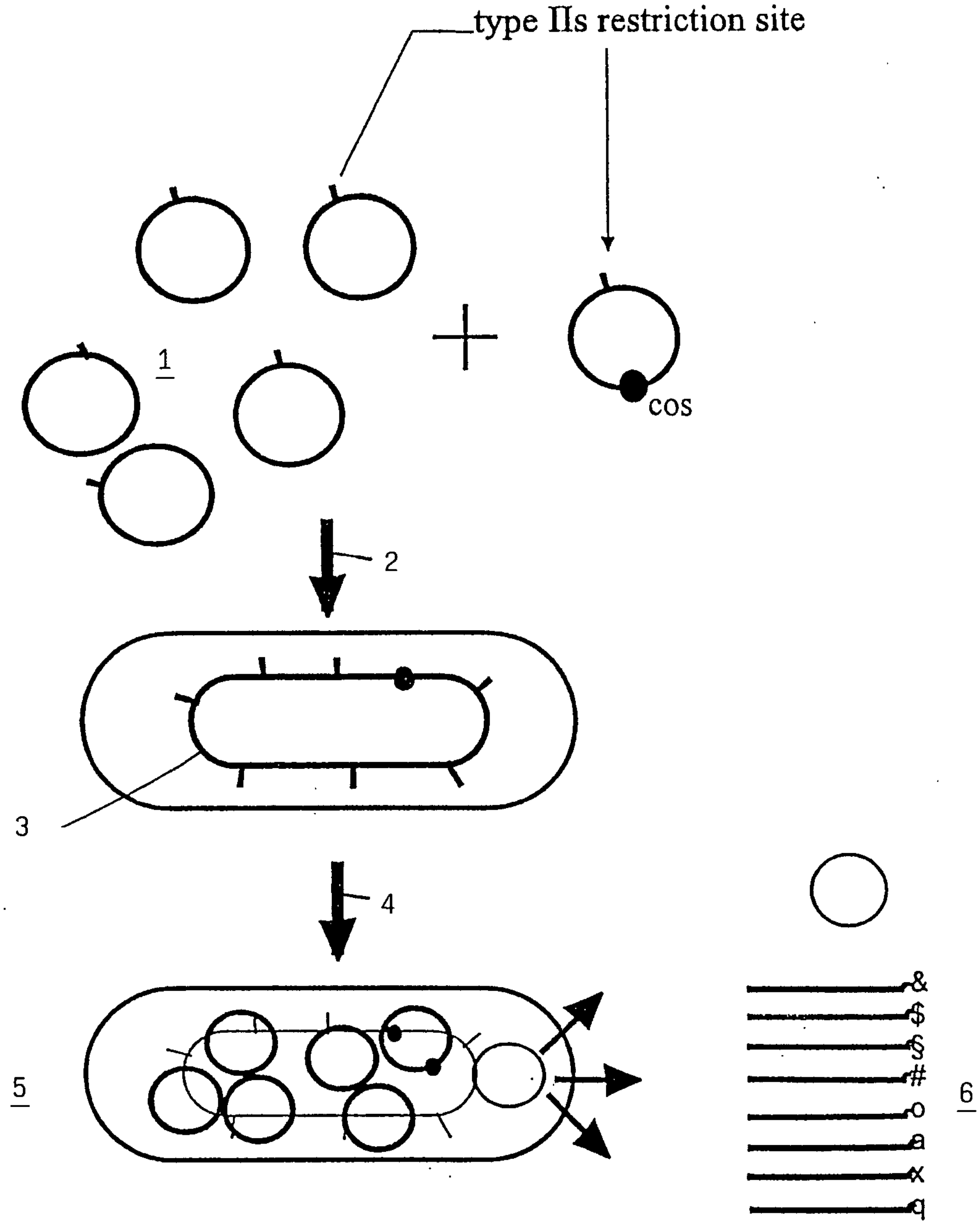

Figure 2.

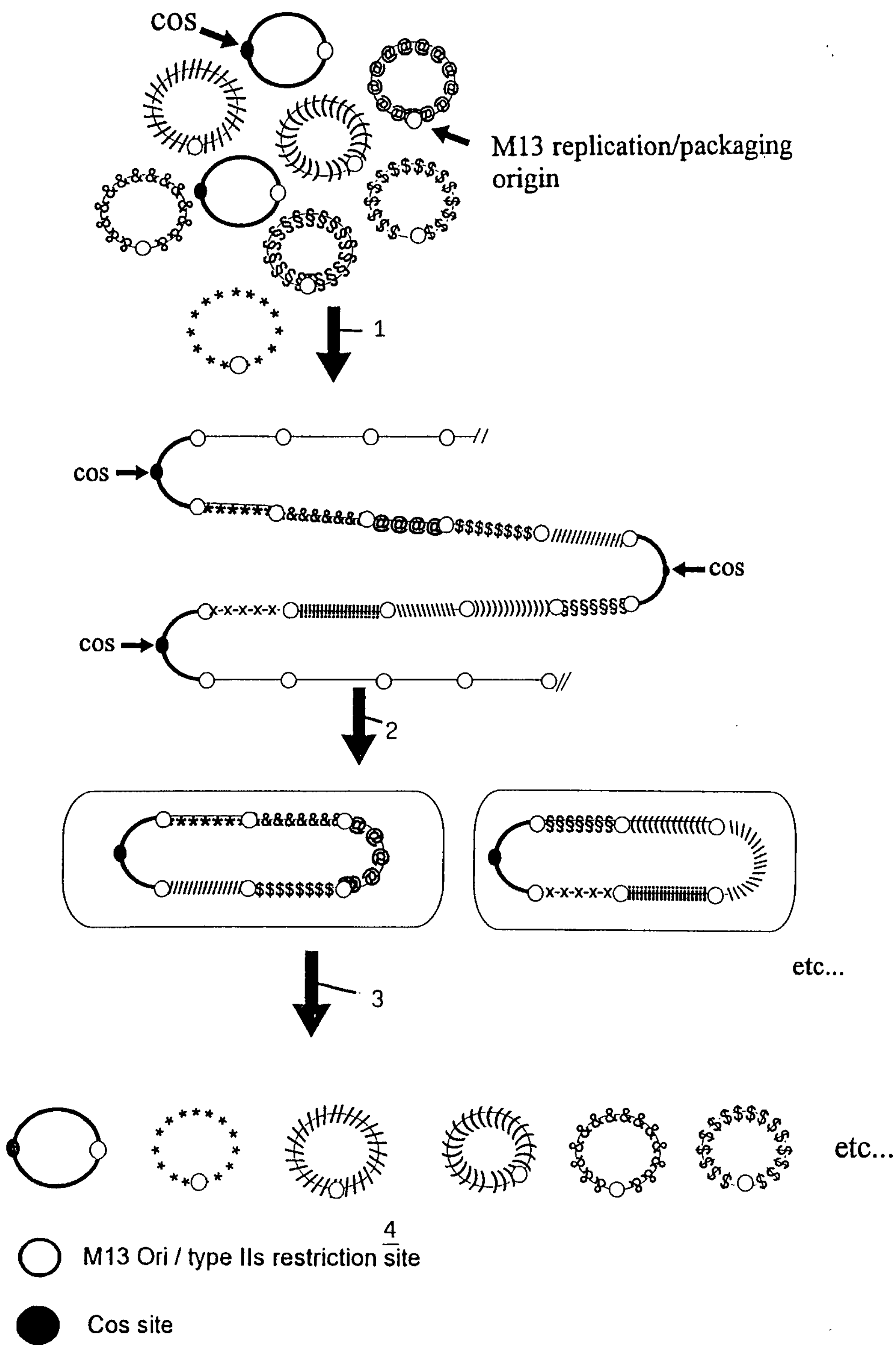
cos
M13 replication/packaging origin
1
cos
cos
cos
2
etc...
3
etc...
4
M13 Ori / type IIs restriction site
Cos site

Figure 3.

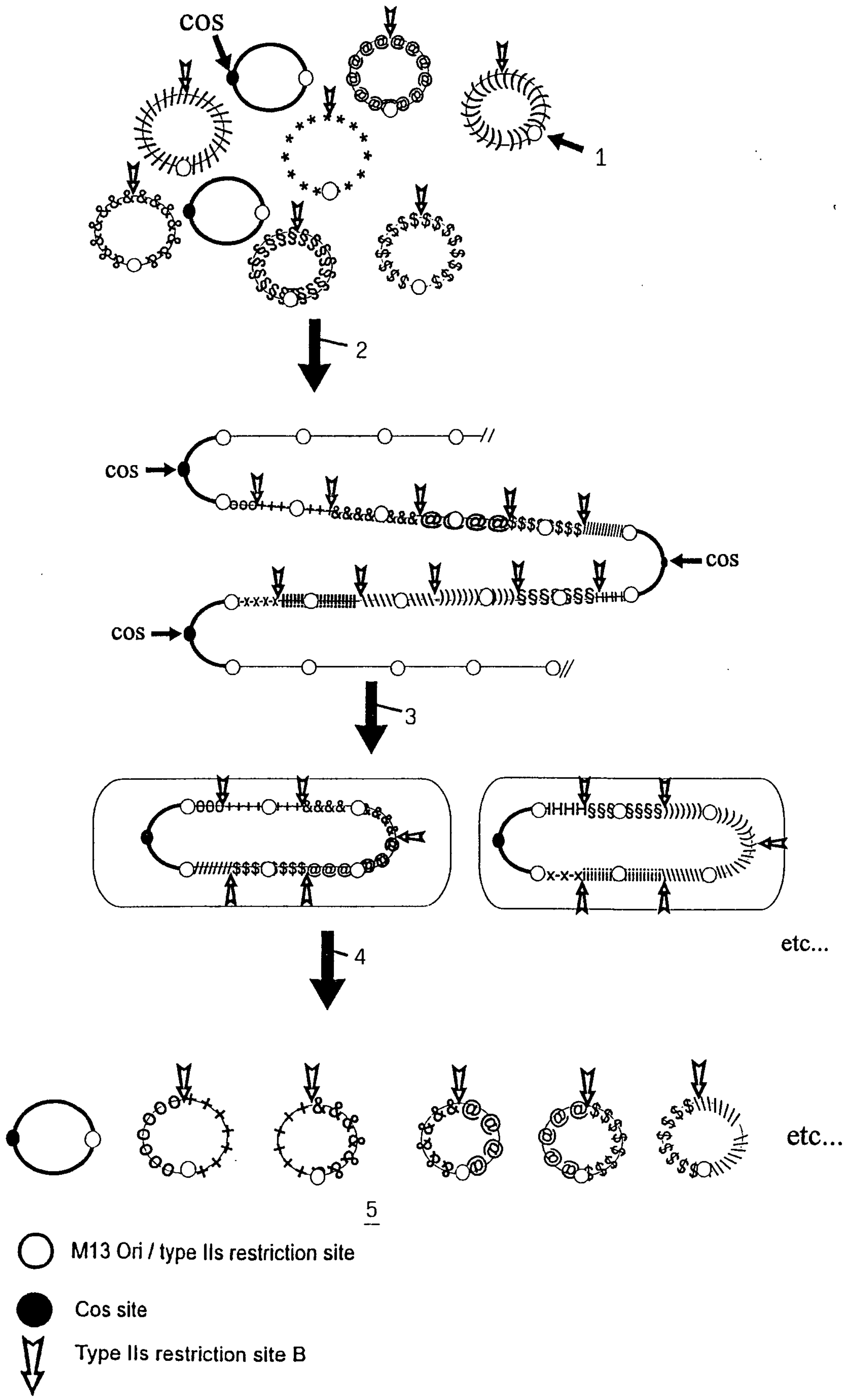
cos
1
2
cos
cos
cos
3
4
etc...
etc...
5
M13 Ori / type IIs restriction site
Cos site
Type IIs restriction site B

Figure 4.

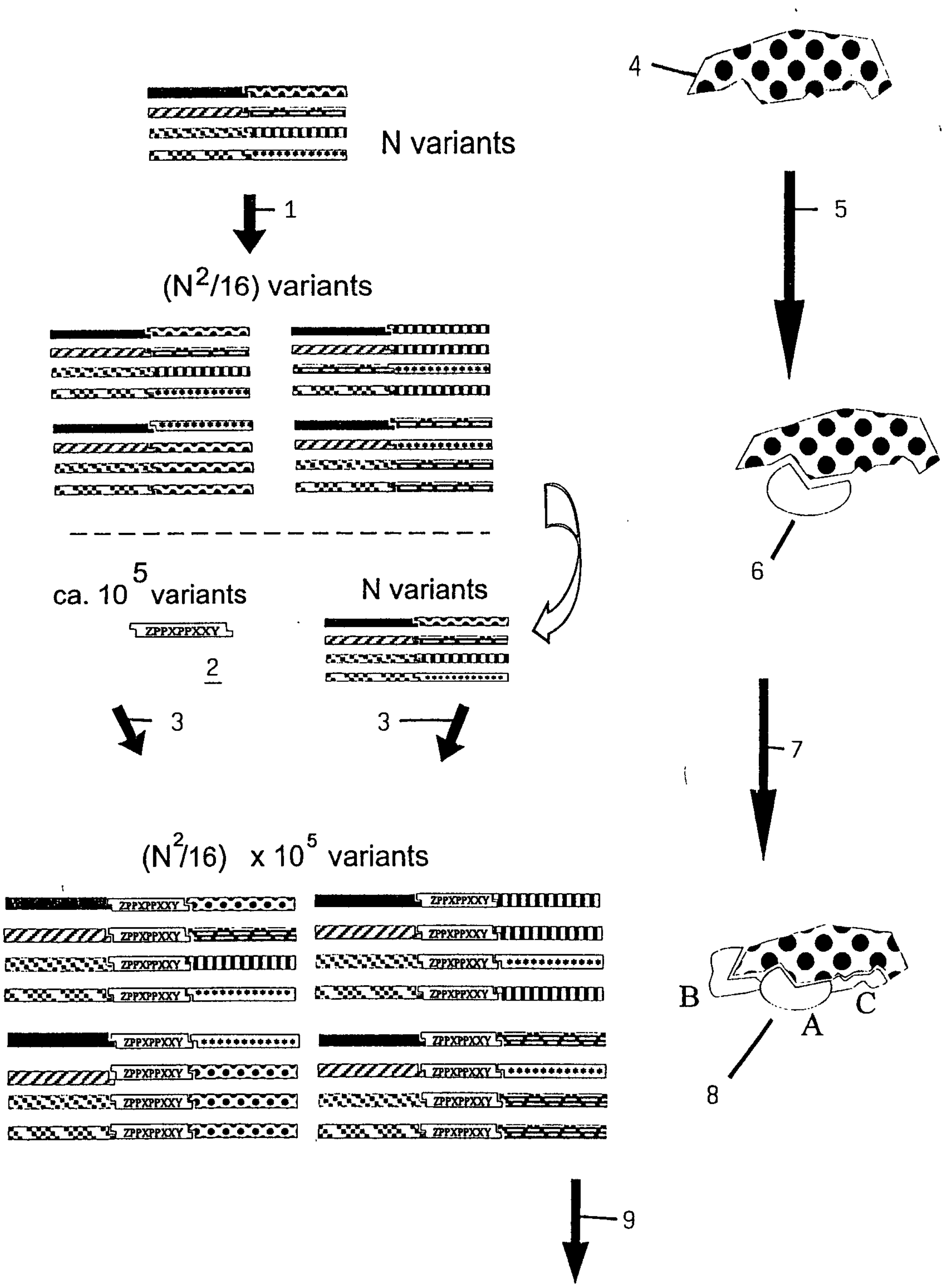

4
N variants
1
5
($N^2$/16) variants
6
ca. $10^5$ variants
N variants
ZPPXPPXXY
2
3
3
7
($N^2$/16) x $10^5$ variants
B
A
C
8
9

Figure 5

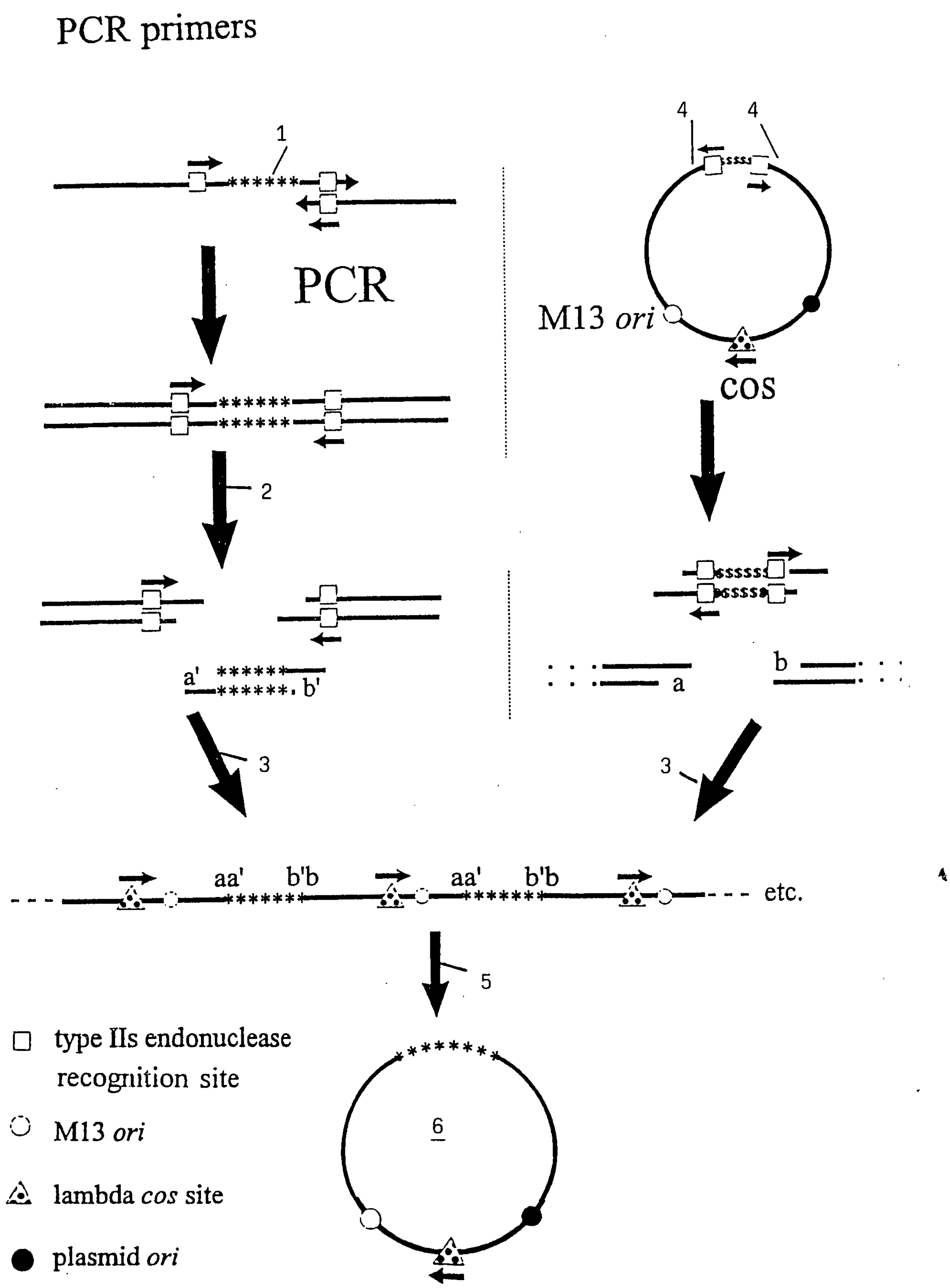
PCR primers
1
PCR
2
a'
b'
3
4
4
M13 ori
cos
a
b
3
aa'
b'b
aa'
b'b
etc.
5
6
type IIs endonuclease recognition site
M13 ori
lambda cos site
plasmid ori

FIGURE 6A

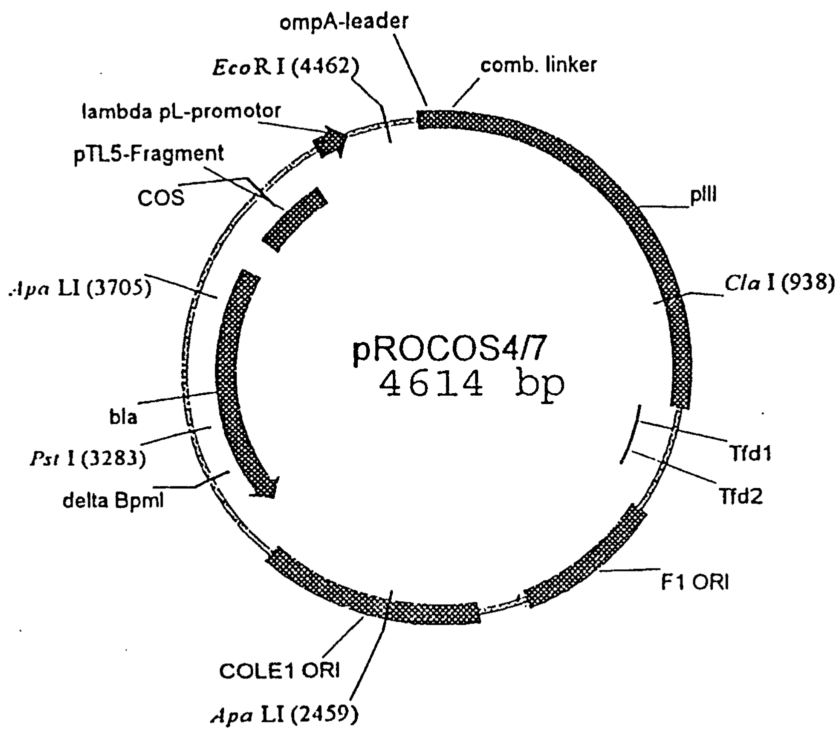
ompA-leader
EcoR I (4462)
comb. linker
lambda pL-promotor
pTL5-Fragment
COS
pIII
Apa LI (3705)
Cla I (938)
pROCOS4/7
4614 bp
bla
Pst I (3283)
Tfd1
delta BpmI
Tfd2
F1 ORI
COLE1 ORI
Apa LI (2459)

1 ggcgagctcc cgtgcagcgc tccaggtacc ccgatatcag agctgaaact gttgaaagtt
61 gtttagcaaa atcccataca gaaaattcat ttactaacgt ctggaaagac gacaaaactt
121 tagatcgtta cgctaactat gagggctgtc tgtggaatgc tacaggcgtt gtagtttgta
181 ctggtgacga aactcagtgt tacggtacat gggttcctat tgggcttgct atccctgaaa
241 atgagggtgg tggctctgag ggtggcggtt ctgagggtgg cggttctgag ggtggcggta
301 ctaaacctcc tgagtacggt gatacaccta ttccgggcta tacttatatc aaccctctcg
361 acggcactta tccgcctggt actgagcaaa accccgctaa tcctaatcct tctcttgagg
421 agtctcagcc tcttaatact ttcatgtttc agaataatag gttccgaaat aggcaggggg
481 cattaactgt ttatacgggc actgttactc aaggcactga ccccgttaaa acttattacc
541 agtacactcc tgtatcatca aaagccatgt atgacgctta ctggaacggt aaattcagag
601 actgcgcttt ccattctggc tttaatgaag atccattcgt ttgtgaatat caaggccaat
661 cgtctgacct gcctcaacct cctgtcaatg ctggcggcgg ctctggtggt ggttctggtg
721 gcggctctga gggtggtggc tctgagggtg gcggttctga gggtggcggc tctgagggag
781 gcggttccgg tggtggctct ggttccggtg attttgatta tgaaaagatg gcaaacgcta
841 ataagggggc tatgaccgaa aatgccgatg aaaacgcgct acagtctgac gctaaaggca
901 aacttgattc tgtcgctact gattacggtg ctgctatcga tggtttcatt ggtgacgttt
961 ccggccttgc taatggtaat ggtgctactg gtgattttgc tggctctaat tcccaaatgg
1021 ctcaagtcgg tgacggtgat aattcacctt taatgaataa tttccgtcaa tatttacctt
1081 ccctccctca atcggttgaa tgtcgccctt ttgtctttgg cgctggtaaa ccatatgaat
1141 tttctattga ttgtgacaaa ataaacttat tccgtggtgt ctttgcgttt cttttatatg
1201 ttgccacctt tatgtatgta ttttctacgt ttgctaacat actgcgtaat aaggagtctt
1261 aatgactcta gaggtcgaaa ttcacctcga aagcaagctg ataaaccgat acaattaaag
1321 gctccttttg gagccttttt ttttggagat tttcaacgtg aaaaaattat tattcgcaat
1381 tccaagctaa ttcacctcga aagcaagctg ataaaccgat acaattaaag gctccttttg
1441 gagccttttt ttttggagat tttcaacgtg aaaaaattat tattcgcaat tccaagctct
1501 gcctcgcgcg tttcggtgat gacggtgaaa acctctgaca catgcagctc ccggagacgg
1561 tcacagcttg tctgtaagcg gatgcagatc acgcgccctg tagcggcgca ttaagcgcgg
1621 cgggtgtggt ggttacgcgc agcgtgaccg ctacacttgc cagcgcccta gcgcccgctc
1681 ctttcgcttt cttcccttcc tttctcgcca cgttcgccag ctttccccgt caagctctaa
1741 atcgggggct ccctttaggg ttccgattta gtgctttacg gcacctcgac cccaaaaaac
1801 ttgattaggg tgatggttca cgtagtgggc catcgccctg atagacggtt tttcgccctt
1861 tgacgttgga gtccacgttc tttaatagtg gactcttgtt ccaaactgga acaacactca
1921 accctatctc ggtctattct tttgatttat aagggatttt gccgatttcg gcctattggt
1981 taaaaaatga gctgatttaa caaaatttta acgcgaattt taacaaaata ttaacgttta
2041 caatttgatc tgcgctcggt cgttcggctg cggcgagcgg tatcagctca ctcaaaggcg
2101 gtaatacggt tatccacaga atcaggggat aacgcaggaa agaacatgtg agcaaaaggc
2161 cagcaaaagg ccaggaaccg taaaaaggcc gcgttgctgg cgtttttcca taggctccgc
2221 ccccctgacg agcatcacaa aaatcgacgc tcaagtcaga ggtggcgaaa cccgacagga
2281 ctataaagat accaggcgtt tccccctgga agctccctcg tgcgctctcc tgttccgacc
2341 ctgccgctta ccggatacct gtccgccttt ctcccttcgg gaagcgtggc gctttctcaa
2401 tgctcacgct gtaggtatct cagttcggtg taggtcgttc gctccaagct gggctgtgtg
2461 cacgaacccc ccgttcagcc cgaccgctgc gccttatccg gtaactatcg tcttgagtcc
2521 aacccggtaa gacacgactt atcgccactg gcagcagcca ctggtaacag gattagcaga
2581 gcgaggtatg taggcggtgc tacagagttc ttgaagtggt ggcctaacta cggctacact
2641 agaaggacag tatttggtat ctgcgctctg ctgaagccag ttaccttcgg aaaaagagtt
2701 ggtagctctt gatccggcaa acaaaccacc gctggtagcg gtggtttttt tgtttgcaag
2761 cagcagatta cgcgcagaaa aaaaggatct caagaagatc ctttgatctt ttctacgggg
2821 tctgacgctc agtggaacga aaactcacgt taagggattt tggtcatgag attatcaaaa
2881 aggatcttca cctagatcct tttaaattaa aaatgaagtt ttaaatcaat ctaaagtata
2941 tatgagtaaa cttggtctga cagttaccaa tgcttaatca gtgaggcacc tatctcagcg
3001 atctgtctat ttcgttcatc catagttgcc tgactccccg tcgtgtagat aactacgata
3061 cgggagggct taccatctgg ccccagtgct gcaatgatac cgcgagaccc acgctcaccg
3121 gctccgcttt tatcagcaat aaaccagcca gccggaaggg ccgagcgcag aagtggtcct
3181 gcaactttat ccgcctccat ccagtctatt aattgttgcc gggaagctag agtaagtagt
3241 tcgccagtta atagtttgcg caacgttgtt gccattgctg caggcatcgt ggtgtcacgc
3301 tcgtcgtttg gtatggcttc attcagctcc ggttcccaac gatcaaggcg agttacatga
3361 tcccccatgt tgtgcaaaaa agcggttagc tccttcggtc ctccgatcgt tgtcagaagt
3421 aagttggccg cagtgttatc actcatggtt atggcagcac tgcataattc tcttactgtc
3481 atgccatccg taagatgctt ttctgtgact ggtgagtact caaccaagtc attctgagaa

FIGURE 6B

```
3541 tagtgtatgc ggcgaccgag ttgctcttgc ccggcgtcaa cacgggataa taccgcgcca
3601 catagcagaa ctttaaaagt gctcatcatt ggaaaacgtt cttcggggcg aaaactctca
3661 aggatcttac cgctgttgag atccagttcg atgtaaccca ctcgtgcacc caactgatct
3721 tcagcatctt ttactttcac cagcgtttct gggtgagcaa aaacaggaag gcaaaatgcc
3781 gcaaaaaagg gaataagggc gacacggaaa tgttgaatac tcatactctt cctttttcaa
3841 tattattgaa gcagacagtt ttattgttca tgatgatata tttttatctt gtgcaatgta
3901 acatcagaga ttttgagaca caacagatct ggccatcatg atggaatgtt tccccggtgg
3961 tgttatctgg cagcagtgcc gtcgatagta tgcaattgat aattattatc atttgcgggt
4021 cctttccggc gatccgcctt gttacggggc ggcgacctcg cgggttttcg ctatttatga
4081 aaatttccg gtttaaggcg tttccgttct tcttcgtcat aacttaatgt ttttatttaa
4141 aataccctct gaaaagaaag gaaacgacag gtgctgaaag cgagcttttt ggccacgatg
4201 cgtccggcgt agaggatctc tcacctacca aacaatgccc ccctgcaaaa aataaattca
4261 tataaaaaac atacagataa ccatctgcgg tgataaatta tctctggcgg tgttgacata
4321 aataccactg gcggtgatac tgagcacatc agcaggacgc actgaccacc atgaaggtga
4381 cgctcttaaa attaagccct gaagaagggc agcattcaaa gcagaaggct ttggggtgtg
4441 tgatacgaaa cgaagcattg gaattctaca acttgcttgg attcctacaa agaagcagca
4501 attttcagtg tcagaagtcg accaaggagg tctagataac gagggcaaaa aatgaaaaag
4561 acagctatcg cgattgcagt ggcactggct ggtttcgcta ccgtagcgca ggcc
```

FIGURE 7A

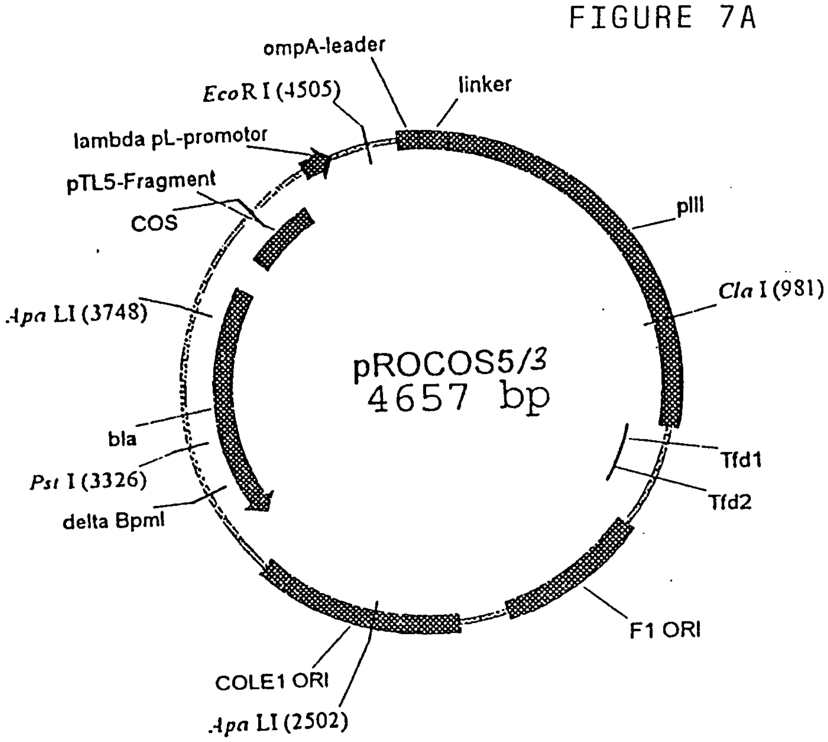

ompA-leader
EcoR I (4505)
linker
lambda pL-promotor
pTL5-Fragment
COS
pIII
Cla I (981)
ApaLI (3748)
pROCOS5/3
4657 bp
bla
Pst I (3326)
Tfd1
Tfd2
delta BpmI
F1 ORI
COLE1 ORI
ApaLI (2502)

FIGURE 7B

```
   1 ggcgagctcc cgtgcagcgg tcttcagcgc ttgccgtctg accgtagcgc tggaagacgc
  61 tccagagggt accccgatat cagagctgaa actgttgaaa gttgtttagc aaaatcccat
 121 acagaaaatt catttactaa cgtctggaaa gacgacaaaa ctttagatcg ttacgctaac
 181 tatgagggct gtctgtggaa tgctacaggc gttgtagttt gtactggtga cgaaactcag
 241 tgttacggta catgggttcc tattgggctt gctatccctg aaaatgaggg tggtggctct
 301 gagggtggcg gttctgaggg tggcggttct gagggtggcg gtactaaacc tcctgagtac
 361 ggtgatacac ctattccggg ctatacttat atcaaccctc tcgacggcac ttatccgcct
 421 ggtactgagc aaaaccccgc taatcctaat ccttctcttg aggagtctca gcctcttaat
 481 actttcatgt ttcagaataa taggttccga aataggcagg gggcattaac tgtttatacg
 541 ggcactgtta ctcaaggcac tgaccccgtt aaaacttatt accagtacac tcctgtatca
 601 tcaaaagcca tgtatgacgc ttactggaac ggtaaattca gagactgcgc tttccattct
 661 ggctttaatg aagatccatt cgtttgtgaa tatcaaggcc aatcgtctga cctgcctcaa
 721 cctcctgtca atgctggcgg cggctctggt ggtggttctg gtggcggctc tgagggtggt
 781 ggctctgagg gtggcggttc tgagggtggc ggctctgagg gaggcggttc cggtggtggc
 841 tctggttccg gtgattttga ttatgaaaag atggcaaacg ctaataaggg ggctatgacc
 901 gaaaatgccg atgaaaacgc gctacagtct gacgctaaag gcaaacttga ttctgtcgct
 961 actgattacg gtgctgctat cgatggtttc attggtgacg tttccggcct tgctaatggt
1021 aatggtgcta ctggtgattt tgctggctct aattcccaaa tggctcaagt cggtgacggt
1081 gataattcac ctttaatgaa taatttccgt caatatttac cttccctccc tcaatcggtt
1141 gaatgtcgcc cttttgtctt tggcgctggt aaaccatatg aattttctat tgattgtgac
1201 aaaataaact tattccgtgg tgtctttgcg tttcttttat atgttgccac ctttatgtat
1261 gtattttcta cgtttgctaa catactgcgt aataaggagt cttaatgact ctagaggtcg
1321 aaattcacct cgaaagcaag ctgataaacc gatacaatta aaggctcctt ttggagcctt
1381 tttttttgga gattttcaac gtgaaaaaat tattattcgc aattccaagc taattcacct
1441 cgaaagcaag ctgataaacc gatacaatta aaggctcctt ttggagcctt tttttttgga
1501 gattttcaac gtgaaaaaat tattattcgc aattccaagc tctgcctcgc gcgtttcggt
1561 gatgacggtg aaaacctctg acacatgcag ctcccggaga cggtcacagc ttgtctgtaa
1621 gcggatgcag atcacgcgcc ctgtagcggc gcattaagcg cggcgggtgt ggtggttacg
1681 cgcagcgtga ccgctacact tgccagcgcc ctagcgcccg ctcctttcgc tttcttccct
1741 tcctttctcg ccacgttcgc cagctttccc cgtcaagctc taaatcgggg gctcccttta
1801 gggttccgat ttagtgcttt acggcacctc gaccccaaaa aacttgatta gggtgatggt
1861 tcacgtagtg ggccatcgcc ctgatagacg gtttttcgcc ctttgacgtt ggagtccacg
1921 ttctttaata gtggactctt gttccaaact ggaacaacac tcaaccctat ctcggtctat
1981 tcttttgatt tataagggat tttgccgatt tcggcctatt ggttaaaaaa tgagctgatt
2041 taacaaaaat ttaacgcgaa ttttaacaaa atattaacgt ttacaatttg atctgcgctc
2101 ggtcgttcgg ctgcggcgag cggtatcagc tcactcaaag gcggtaatac ggttatccac
2161 agaatcaggg gataacgcag gaaagaacat gtgagcaaaa ggccagcaaa aggccaggaa
2221 ccgtaaaaag gccgcgttgc tggcgttttt ccataggctc cgcccccctg acgagcatca
2281 caaaaatcga cgctcaagtc agaggtggcg aaacccgaca ggactataaa gataccaggc
2341 gtttccccct ggaagctccc tcgtgcgctc tcctgttccg accctgccgc ttaccggata
2401 cctgtccgcc tttctccctt cgggaagcgt ggcgctttct caatgctcac gctgtaggta
2461 tctcagttcg gtgtaggtcg ttcgctccaa gctgggctgt gtgcacgaac cccccgttca
2521 gcccgaccgc tgcgccttat ccggtaacta tcgtcttgag tccaacccgg taagacacga
2581 cttatcgcca ctggcagcag ccactggtaa caggattagc agagcgaggt atgtaggcgg
2641 tgctacagag ttcttgaagt ggtggcctaa ctacggctac actagaagga cagtatttgg
2701 tatctgcgct ctgctgaagc cagttacctt cggaaaaaga gttggtagct cttgatccgg
2761 caaacaaacc accgctggta gcggtggttt ttttgtttgc aagcagcaga ttacgcgcag
2821 aaaaaaagga tctcaagaag atcctttgat cttttctacg gggtctgacg ctcagtggaa
2881 cgaaaactca cgttaaggga ttttggtcat gagattatca aaaaggatct tcacctagat
2941 ccttttaaat taaaaatgaa gttttaaatc aatctaaagt atatatgagt aaacttggtc
3001 tgacagttac caatgcttaa tcagtgaggc acctatctca gcgatctgtc tatttcgttc
3061 atccatagtt gcctgactcc ccgtcgtgta gataactacg atacgggagg gcttaccatc
3121 tggccccagt gctgcaatga taccgcgaga cccacgctca ccggctccgc ttttatcagc
3181 aataaaccag ccagccggaa gggccgagcg cagaagtggt cctgcaactt tatccgcctc
3241 catccagtct attaattgtt gccgggaagc tagagtaagt agttcgccag ttaatagttt
3301 gcgcaacgtt gttgccattg ctgcaggcat cgtggtgtca cgctcgtcgt ttggtatggc
3361 ttcattcagc tccggttccc aacgatcaag gcgagttaca tgatccccca tgttgtgcaa
3421 aaaagcggtt agctccttcg gtcctccgat cgttgtcaga agtaagttgg ccgcagtgtt
3481 atcactcatg gttatggcag cactgcataa ttctcttact gtcatgccat ccgtaagatg
```

still FIGURE 7B

```
3541 cttttctgtg actggtgagt actcaaccaa gtcattctga gaatagtgta tgcggcgacc
3601 gagttgctct tgcccggcgt caacacggga taataccgcg ccacatagca gaactttaaa
3661 agtgctcatc attggaaaac gttcttcggg gcgaaaactc tcaaggatct taccgctgtt
3721 gagatccagt tcgatgtaac ccactcgtgc acccaactga tcttcagcat cttttacttt
3781 caccagcgtt tctgggtgag caaaaacagg aaggcaaaat gccgcaaaaa agggaataag
3841 ggcgacacgg aaatgttgaa tactcatact cttccttttt caatattatt gaagcagaca
3901 gttttattgt tcatgatgat atatttttat cttgtgcaat gtaacatcag agattttgag
3961 acacaacaga tctggccatc atgatggaat gtttccccgg tggtgttatc tggcagcagt
4021 gccgtcgata gtatgcaatt gataattatt atcatttgcg ggtcctttcc ggcgatccgc
4081 cttgttacgg ggcggcgacc tcgcgggttt tcgctattta tgaaaatttt ccggtttaag
4141 gcgtttccgt tcttcttcgt cataacttaa tgtttttatt taaaataccc tctgaaaaga
4201 aaggaaacga caggtgctga aagcgagctt tttggccacg atgcgtccgg cgtagaggat
4261 ctctcaccta ccaaacaatg ccccccctgca aaaaataaat tcatataaaa aacatacaga
4321 taaccatctg cggtgataaa ttatctctgg cggtgttgac ataaatacca ctggcggtga
4381 tactgagcac atcagcagga cgcactgacc accatgaagg tgacgctctt aaaattaagc
4441 cctgaagaag ggcagcattc aaagcagaag gctttggggt gtgtgatacg aaacgaagca
4501 ttggaattct acaacttgct tggattccta caaagaagca gcaattttca gtgtcagaag
4561 tcgaccaagg aggtctagat aacgagggca aaaaatgaaa aagacagcta tcgcgattgc
4621 agtggcactg gctggtttcg ctaccgtagc gcaggcc
```